(19) Europäisches Patentamt
European Patent Office
Office européen des brevets

(11) EP 3 636 638 A1

(12) EUROPÄISCHE PATENTANMELDUNG

(43) Veröffentlichungstag:
**15.04.2020 Patentblatt 2020/16**

(51) Int Cl.:
***C07D 233/58*** *(2006.01)*

(21) Anmeldenummer: **18199151.4**

(22) Anmeldetag: **08.10.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **proionic GmbH**
**8074 Grambach (AT)**

(72) Erfinder:
- **KALB, Roland**
**8074 Grambach (AT)**
- **DAMM, Markus**
**8074 Grambach (AT)**

(74) Vertreter: **Schwarz & Partner Patentanwälte OG**
**Patentanwälte**
**Wipplingerstraße 30**
**1010 Wien (AT)**

Bemerkungen:
Ein Antrag gemäss Regel 139 EPÜ auf Berichtigung der Beschreibung liegt vor. Über diesen Antrag wird im Laufe des Verfahrens vor der Prüfungsabteilung eine Entscheidung getroffen (Richtlinien für die Prüfung im EPA, A-V, 3.).

(54) **ZUSAMMENSETZUNG UMFASSEND EINE IONISCHE FLÜSSIGKEIT MIT FLUORIERTEM ANION**

(57) Zusammensetzung umfassend eine ionische Flüssigkeit der allgemeinen Formel $[A^+]$ $[B^-]$, wobei $[A^+]$ ein organisches Kation ist und $[B^-]$ ein Anion ist ausgewählt aus $BF_4^-$ und $PF6^-$, wobei die Zusammensetzung ein Additiv umfasst, wobei das Additiv einen $pK_S$-Wert von zumindest 5 aufweist und Verwendungen der Zusammensetzung.

EP 3 636 638 A1

## Beschreibung

Die vorliegende Erfindung betrifft eine Zusammensetzung umfassend eine ionische Flüssigkeit der allgemeinen Formel [$A^+$] [$B^-$], wobei [$A^+$] ein organisches Kation ist und [$B^-$] ein Anion ist ausgewählt aus $BF_4^-$ und $PF_6^-$, sowie Verwendungen davon.

HINTERGRUND DER ERFINDUNG

Ionische Flüssigkeiten sind - im Sinne der anerkannten Literatur (z.B. Wasserscheid, Peter; Welton, Tom (Eds.); "Ionic Liquids in Synthesis", 2nd Edition, Verlag Wiley-VCH 2008, ISBN-13:978-3527312399; MacFarlane, Doug; Kar, Mega; Pringle, Jennifer M.; "Fundamentals of Ionic Liquids: From Chemistry to Applications", Verlag Wiley-VCH 2017, ISBN-13:978-3527339990) organische Salze oder Salzgemische, bestehend aus organischen Kationen und organischen oder anorganischen Anionen, mit Schmelzpunkten von unter 100°C, in ihrem flüssigen Phasenzustand. In diesen Salzen können zusätzlich anorganische Salze gelöst sein und des Weiteren auch molekulare Hilfsstoffe. Im Sinne dieser Anmeldung sehen wir die willkürlich mit 100°C festgelegte Grenze des Schmelzpunktes ionischer Flüssigkeiten in weiterem Sinne und schließen somit auch solche Salzschmelzen ein, die einen Schmelzpunkt von über 100°C, aber unter 200°C haben. Die ionischen Flüssigkeiten mit Schmelzpunkt zwischen 100 °C und 200 °C unterscheiden sich nämlich ansonsten nicht in ihren Eigenschaften von ionischen Flüssigkeit mit niedrigerem Schmelzpunkt. Ionische Flüssigkeiten weisen äußerst interessante Eigenschaften auf, wie beispielsweise einen sehr geringen bis nahezu nicht messbaren Dampfdruck, einen sehr großen Liquidusbereich, gute elektrische Leitfähigkeit und ungewöhnliche Solvatations-Eigenschaften. Diese Eigenschaften prädestinieren sie für den Einsatz in verschiedenen Bereichen technischer Anwendungen. So können sie beispielsweise als Lösungsmittel (bei organischer und anorganischer Synthese im Allgemeinen, bei der Übergangsmetallkatalyse, der Biokatalyse, der Phasentransfer-Katalyse, bei Mehrphasen-Reaktionen, in der Photochemie, in der Polymersynthese und der Nanotechnologie), als Extraktionsmittel (bei der flüssig-flüssig- und der flüssig-gasförmigen-Extraktion im Allgemeinen, der Entschwefelung von Rohöl, der Entfernung von Schwermetallen aus Abwässern, der Flüssigmembranextraktion), als Elektrolyte (in Batterien, Brennstoffzellen, Kondensatoren, Solarzellen, Sensoren, in der Galvanotechnik, in der elektrochemischen Metallbearbeitung, in der elektrochemischen Synthese im Allgemeinen, bei der elektroorganischen Synthese, der Nanotechnologie), als Schmierstoffe, als Thermofluide, als Gele, als Reagenzien zur organischen Synthese, in der "Green Chemistry" (Ersatz für Volatile Organic Compounds), als Antistatika, in Spezialanwendungen der Analytik (Gaschromatographie, Massenspektroskopie, Kapillarzonenelektrophorese), als Flüssigkristalle, etc. eingesetzt werden. Bei der Anwendung von ionischen Flüssigkeiten mag die Optimierung der Eigenschaften für die jeweilige Anwendung in weiten Grenzen durch eine Variation der Struktur von Anion und Kation bzw. eine Variation ihrer Kombination erfolgen, was den ionischen Flüssigkeiten übrigens ganz allgemein die Bezeichnung "Designer Solvents" (siehe beispielsweise Freemantle, M.; Chem. Eng. News, 78, 2000, 37) eingebracht hat. Zum Zeitpunkt der Erstellung dieser Patentanmeldung wurden bisher rund 77.000 wissenschaftliche Publikationen und 15.800 Patentfamilien angemeldet (CAS SciFinder™ Datenbanksuche nach "Ionic liquids" als "concept"), welchen Tausende bis Zehntausende verschiedener, experimentell untersuchter ionischer Flüssigkeiten zugeordnet sind, sowie Millionen theoretischer Strukturen.

Ein großer Teil der in der Literatur beschriebenen ionischen Flüssigkeiten weist fluorierte Anionen auf, z.B. Tetrafluoroborat ($BF_4^-$, rund 9.000 Publikationen), Hexafluorophosphat ($PF_6^-$, rund 8.000 Publikationen), Triflat ($CF_3SO_3^-$, rund 5.000 Publikationen), Bistriflylamid ($CF_3$-$SO_2$-$N^-$-$SO_2$-$CF_3$, rund 13.500 Publikationen) oder Trifluoracetat ($CF_3CO_2^-$, rund 1.000 Publikationen). Dies ist vor allem darauf zurückzuführen, dass fluorierte ionische Flüssigkeiten (fluorinated ionic liquids, "FILs") allgemein deutlich höhere thermische und elektrochemische Stabilität, wesentlich geringere Viskosität, deutlich niedrigere Schmelzpunkte und bessere Schmiereigenschaften als ihre nichtfluorierten Pendants aufweisen (Xue, Hong; Verma, Rajendar; Shreeve, Jean'ne M.; "Review of ionic liquids with fluorine-containing anions", Journal of Fluorine Chemistry 127 (2006), 159-176). Viele dieser FILs sind außerdem hydrophob bis superhydrophob, einige sogar hydrophob und lipophob gleichzeitig; im Gegensatz dazu sind die meisten nichtfluorierten ionischen Flüssigkeiten vollkommen wassermischbar und meist (stark) hygroskopisch, ausgenommen solche mit langen Seitenketten am Kation oder Anion, welche dann aber wiederum hohe Viskosität und geringe thermische Stabilität zeigen und sich ähnlich wie Detergenzien verhalten können. Um ionische Flüssigkeiten z.B. in der flüssig-flüssig Extraktion mit wässrigen Medien benützen zu können, sind daher FILs auch hier klar im Vorteil. Aufgrund der genannten Vorteile, werden FILs daher z.B.

- als Elektrolyte (in Superkondensatoren, Batterien, Farbstoffsolarzellen, Brennstoffzellen, Thermoelektrischen Zellen; siehe z.B. MacFarlane et al., Energy Environ. Sci., 2014, 7, 232),
- als Wärmeträger- und Wärmespeichermedien (siehe z.B. MacFarlane et al., Energy Environ. Sci., 2014, 7, 232; Kalb, Roland; WO2013113461; Filzwieser, Iris und Filzwieser Andreas; WO2010136403; Chernikova, E. A et al.; Russian Chemical Reviews (2015), 84(8), 875-890) und

- als rezyklierbare Lösungsmittel in der organischen Synthese, der Katalyse und der chemischen oder thermischen Trenntechnik (Xue, Hong; Verma, Rajendar; Shreeve, Jean'ne M.; "Review of ionic liquids with fluorine-containing anions", Journal of Fluorine Chemistry 127 (2006), 159-176; Meyer, Carolin; Werner, Sebastian; Haumann, Marco; Wasserscheid, Peter; edited by Plechkova, Natalia; Seddon, Kenneth; Ionic Liquids Completely UnCOILed (2015), ISBN-13: 978-1-118-84009-2) verwendet.

Zu den Nachteilen der FILs zählen der i.A. hohe Preis, welcher erwartungsgemäß aus den hohen Kosten der fluorierten Ausgangsstoffe ihrer Synthese (z.B. Trifluormethansulfonsäure, Trifluoressigsäure oder Bis-(trifluormethylsulfonyl)amid) resultiert, und die z.T. erhöhte Toxizität (Steudte, Stephanie; Stolte, Stefan et al., Chem. Commun. (2012), 48, 9382-9384), welche in vielen Fällen mit der Bildung von giftigem Fluorwasserstoff im Zusammenhang steht.

Bezüglich der Kosten der FILs gibt es allerdings zwei Anionen, nämlich das Anion Tetrafluoroborat ($BF_4^-$) und das Anion Hexafluorophosphat ($PF_6^-$), deren Precursor deutlich günstiger in großtechnischen Mengen verfügbar sind. Die Precursor sind ihre $Na^+$, $K^+$ oder $NH_4^+$-Salze und insbesondere die konjugierten Säuren Tetrafluoroborsäure ($HBF_4$) und Hexafluorophosphorsäure ($HPF_6$). Diese Säuren kommen dabei als 45-50%ige bzw. 60-70%ige wässrige Lösung in den Handel. Sowohl Hexafluorophosphorsäure als auch Tetrafluoroborsäure sind sehr starke Brønstedsäuren und liegen in wässriger Lösung nahezu vollständig dissoziiert als $H_3O^+$ $BF_4^-$ bzw. $H_3O^+$ $PF_6^-$ vor.

Ionische Flüssigkeiten basierend auf diesen beiden Anionen zählen zu den allerersten FILs, welche bereits in den Jahren 1992 (1-Ethyl-3-methylimidazolium tetrafluoroborat; Wilkes, John S.; Zaworotko, Michael J.; Journal of the Chemical Society, Chemical Communications (1992), (13), 965-7) und 1994 (1-Ethyl-3-Methylimidazolium Hexafluorophosphate; Fuller, Joan; Carlin, Richard T.; De Long, Hugh C.; Haworth, Dustin; Journal of the Chemical Society, Chemical Communications (1994), (3), 299-300) publiziert worden sind und damals als "air and water stable" eingestuft worden sind, was sich später als Irrtum herausstellen sollte.

Im Laufe der Zeit erschienen tausende wissenschaftliche Publikationen, welche auf FILs mit diesen beiden Anionen basieren und die Anzahl pro Jahr steigerte sich bis 2012 (Fig. 1). Figur 1 zeigt die chronologische Entwicklung der Anzahl der publizierten Literaturstellen und Patente mit $BF_4^-$ und $PF_6^-$ basierenden FILs (CAS SciFinder™ "concept" search). Ab 2012 ist deutlich zu sehen, dass die Anzahl der Literaturstellen stark zurückgeht und bis 2017 auf das Niveau von 2006 sinkt; ein ähnliches Bild zeigt sich bei den Patenten, die eine Untergruppe der Literaturstellen sind. Dies ist vermutlich darauf zurückzuführen, dass das Bewusstsein für die mangelnde hydrolytische Stabilität von $BF_4^-$ und $PF_6^-$ in Fachkreisen deutlich gestiegen war, auf internationalen Kongressen von führenden Wissenschaftlern kommuniziert wurde und einige Leitartikel zu dieser Thematik publiziert wurden (siehe z.B. "Hydrolysis study of fluoroorganic and cyano-based ionic liquid anions - consequences for operational safety and environmental stability"; Steudte, Stephanie; Stolte, Stefan et al., Green Chem. (2012), 14, 2472; "Hydrolysis of Tetrafluoroborate and Hexafluorophosphate Counter Ions in Imidazolium-Based Ionic Liquids"; Freire, Mara G.; Coutinho, Joao A. P. et al., J. Phys. Chem. A (2010), 114, 3744-3749.).

Bei der Hydrolyse $BF_4^-$ und $PF_6^-$ entstehen Fluoridanionen $F^-$, die bekanntermaßen in wässriger Lösung - in Abhängigkeit vom pH-Wert - im Gleichgewicht mit Fluorwasserstoff (HF) vorliegen, welches als Gas freigesetzt werden kann:

$$F^- + H_3O^+ \rightleftarrows H_2O + HF$$

Fluorwasserstoff ist giftig. Dies wird beispielsweise reflektiert in einem Arbeitsplatzgrenzwert von 1,8 ppm (8-Stunden-Mittelwert) gemäß Richtlinie 2000/39/EG. Im Vergleich dazu liegt der 8-Stunden-Mittelwert für Cyanwasserstoff bei 0,9 ppm (Daten aus GESTIS-Stoffdatenbank des Instituts für Arbeitsschutz der Deutschen Gesetzlichen Unfallversicherung, www.dguv.de/ifa/stoffdatenbank).

Die Gefahr der unkontrollierten Entwicklung von sehr giftigem Fluorwasserstoff aus $BF_4^-$ und $PF_6^-$ basierenden FILs wurde, wie durch den Rückgang der entsprechenden Publikationen (Fig. 1) belegt, erkannt. Die Freisetzung von HF ist bis heute ein ungelöstes Problem, sodass diese ionischen Flüssigkeiten, insbesondere bei Kontakt mit Wasser bzw. Alkoholen, trotz ihrer hervorragenden Eigenschaften und des vergleichsweise geringen Preises gegenüber anderen FILs, in vielen Anwendungen technisch ungeeignet sind. Auch die wissenschaftlichen Analysen erscheinen problematisch, da mit diesen FILs assoziierte Effekte möglicherweise durch freigesetztes HF bedingt sind.

Entsprechend stellt sich die Aufgabe, eine ionische Flüssigkeit mit den Anionen $BF_4^-$ und/oder $PF_6^-$ derart bereitzustellen, dass ihre Benutzung auch im Kontakt mit Wasser bzw. Alkoholen sicher möglich ist und dabei ihre Verwendungsmöglichkeiten nicht einzuschränken.

BESCHREIBUNG DER ERFINDUNG

Die Aufgabe wird gelöst durch eine Zusammensetzung, umfassend eine ionische Flüssigkeit der allgemeinen Formel $[A^+][B^-]$, wobei $[A^+]$ ein organisches Kation ist und $[B^-]$ ein Anion ist ausgewählt aus $BF_4^-$ und $PF_6^-$, und wobei

die Zusammensetzung ein Additiv umfasst, wobei das Additiv einen $pK_S$-Wert von zumindest 5 aufweist.

Es konnte gezeigt werden, dass durch die erfindungsgemäße Zusammensetzung die Freisetzung von Fluorwasserstoff (HF) unterbunden wird, wenn die Zusammensetzung in Kontakt mit Wasser oder Alkoholen kommt.

Additive, die einem $pK_S$-Wert von zumindest 5 aufweisen, sind, wie in den Beispielen gezeigt, geeignet, den pH-Wert, der sich dauerhaft einstellt, wenn die ionische Flüssigkeit mit bis zu 50 % Wasser in Kontakt kommt, derart zu beeinflussen, dass eine Freisetzung von Fluorwasserstoff minimiert wird. Der $pK_S$-Wert eines Additives ist die sogenannte Säurekonstante und ergibt sich als der negativ dekadische Logarithmus der Gleichgewichtskonstante $K_S$ (- log $K_S$). Diese beschreibt das Gleichgewicht für die Reaktion der konjugierten Säure des Additives (Additiv$H^+$) mit Wasser zum Additiv und $H_3O^+$:

$$\text{AdditivH}^+ + H_2O \rightleftarrows \text{Additiv} + H_3O^+$$

Der pKs-Wert ist ein dem Fachmann bekannter Parameter. Je höher der pKs-Wert des Additivs desto stärker sind die basischen Eigenschaften ausgeprägt.

Der Zusammenhang zwischen pH-Wert und der möglichen Freisetzung von Fluorwasserstoff ist komplex, da es bei längerem Kontakt mit Wasser zu einem quantitativ nicht vorhersehbaren Abfall des pH-Werts kommt. Dies ist wahrscheinlich bedingt durch die Hydrolyse der Anionen $BF_4^-$ und $PF_6^-$, für die vermutet wird, dass sie nach den folgenden Schritten abläuft:

$$BF_4^- + H_2O \rightleftarrows BF_3OH^- + HF$$

$$BF_3OH^- + 2\ H_2O \rightleftarrows H_2BO_3^- + 3\ HF \text{ (langsam über mehrere Zwischenstufen)}$$

$$PF_6^- + H^+ \rightleftarrows PF_5 + HF$$

$$PF_5 + 4H_2O \rightleftarrows H_3PO_4 + 5\ HF \text{ (über mehrere Zwischenstufen)}$$

An Hand der Mechanismen könnte man vermuten, dass Hydroxidionen die Hydrolyse verstärken, so dass der Fachmann von Additiven mit höheren $pK_S$-Werten Abstand nehmen würde.

Allerdings haben die hier vorgelegten Untersuchungen gezeigt, dass die Additive mit einen $pK_S$-Wert von zumindest 5 einen stabilisierenden Effekt auf die pH-Wert-Einstellung haben, was von entscheidende Bedeutung für die Sicherheit ist. Inwieweit freigesetzte Fluorid als $F^-$ (Salz) bzw. giftige Flusssäure (HF, Säure) vorliegen wird nämlich durch den pH-Wert bestimmt, und kann mittels des $pK_S$-Wert von Flusssäure, der bei 3,17 liegt (Römpp Online 4.0, https://roempp.thieme.de) und der dem Fachmann bekannten Henderson-Hasselbalch-Gleichung berechnet werden:

$$pH = pKs + \log (c_{Salz} / c_{Säure}) \text{ mit } c = \text{Konzentration in mol/l}$$

Folglich:

$$(c_{Salz} / c_{Säure}) = 10^{(pH-pKs)}.$$

In Beispiel 1 wurde die pH-Wert Entwicklung untersucht von Zusammensetzungen der ionischen Flüssigkeit 1-Ethyl-3-methylimidazolium-tetrafluoroborat, die mit 50m% Wasser gemischt wird. Die unbehandelte ionische Flüssigkeit ist bereits kurz nach der Mischung mit Wasser sauer (pH-Wert ~2,5). Auch im Vergleichsbeispiel, bei dem die ionische Flüssigkeit mittels Ionenaustauscher neutralisiert wurde, fällt der pH-Wert von 7,1 auf einen pH-Wert von 3,9. Durch Additive mit $pK_S$-Werten von zumindest 5 in der Zusammensetzung wird hingegen eine Einstellung des pH-Werts in einem höheren Bereich erreicht, wie beispielsweise 4,5 für die vergleichsweise schwache Base Pyridin, auch wenn der anfängliche pH-Wert sogar schon unter 7 liegt.

Gemäß der obigen Gleichung liegen bei einem pH-Wert von 4,4 nur etwa 5,6 % der Fluoridionen als giftiger Fluorwasserstoff vor, während bei einem pH-Wert von etwa 2,5, der für die unbehandelte ionische Flüssigkeit beobachtet wird, etwa 87 % Fluorwasserstoff vorliegen.

Zusätzlich konnte gezeigt werden (Beispiel 10 und 11), dass die Zugabe des Additivs die Eigenschaften der Zusammensetzung im Vergleich zur ionischen Flüssigkeit der allgemeinen Formel $[A^+][B^-]$ als solches wenig beeinflusst oder sogar verbessert. Damit ist die Verwendung beispielsweise als Elektrolyt, Wärmeträger, Kühlmittel möglich, also auch in anspruchsvollen Anwendungen, bei denen eine elektrochemische Stabilität und gute Materialverträglichkeiten gefordert sind.

So sind erfindungsgemäße Zusammensetzungen beispielsweise dadurch ausgezeichnet, dass sie, nach einer thermischen Behandlung, vorzugsweise bei 80 °C, zumindest aber über dem Schmelzpunkt der ionischen Flüssigkeit, nach einer Zeitdauer einen pH50-Wert von über 4 bevorzugt über 5, zeigen, wobei der pH50-Wert, der pH-Wert ist, den die Zusammensetzung oder eine Probe der Zusammensetzung zeigt mit einem Wasseranteil von 50 m% und wobei die Zeitdauer so gewählt ist, dass der pH50-Wert keine weitere Änderung zeigt (Equilibrierung).

Ein ähnlicher Mechanismus bei der Hydrolyse wird für die Alkoholyse/Solvolyse vermutet und tatsächlich zeigen die Additive bei Kontakt mit zumindest einem Alkohol einen vergleichbaren Effekt (Beispiel 5). Als Alkohol wird eine Verbindung der allgemeinen Formel R-OH verstanden, wobei R gemäß der vorliegenden Erfindung vorzugsweise eine Alkylgruppe ist, d.h. Alkohole sind zum Beispiel Ethanol, Methanol, Propanol und isoPropanol.

Die erfindungsgemäßen Zusammensetzungen stellen also eine ionische Flüssigkeit der allgemeinen Formel $[A^+]$ $[B^-]$, wobei $[A^+]$ ein organisches Kation ist und $[B^-]$ ein Anion ist ausgewählt aus $BF_4^-$ und $PF_6^-$, derart bereit, dass eine sichere Verwendung auch dann gewährleistet ist, wenn die Zusammensetzung Wasser oder zumindest einen Alkohol enthält (oder ein Gemisch aus Wasser und/oder Alkoholen) oder in Kontakt mit Wasser oder zumindest einem Alkohol kommen kann (oder einem Gemisch aus Wasser und/oder Alkoholen).

In einer Ausführungsform enthält die Zusammensetzung daher weiters Wasser, zumindest einen Alkohol oder ein Gemisch davon oder kann in Kontakt mit Wasser, einem Alkohol oder ein Gemisch davon kommen. Dabei ist bevorzugt, dass die Zusammensetzung Wasser und/oder Alkohol enthält bis zu maximal 50 m%, bevorzugt bis zu 10 m%, oder bis zu 2 m%, wobei m% für Gewichtsprozent steht und sich auf das Gesamtgewicht der Zusammensetzung bezieht. Auch bevorzugt sind Anwendungen, in denen die Zusammensetzung mit einer entsprechenden Menge an Wasser, Alkohol oder Gemisch davon in Kontakt kommt bzw. kommen kann. Ein Wassergehalt von ungefähr 2 m% kann bereits durch den Kontakt mit feuchter (z.B. warmer) Luft erreicht werden. Die Erfindung betrifft daher auch die Verwendung der Zusammensetzungen und Verfahren, in denen die Zusammensetzung mit Wasser, zumindest einem Alkohol oder einem Gemisch davon in Kontakt kommt.

In einer bevorzugten Ausführungsform ist das Additiv eine Stickstoffbase. Stickstoffbasen können erfindungsgemäß beispielsweise Amine gemäß der generischen Formel $R_3N$ oder andere organische Stickstoffverbindungen gemäß der generischen Formel R=N-R sein, wobei die Reste R unabhängig voneinander gewählt sind und/oder zumindest zwei der Reste mit dem Stickstoffatom, an das sie gebunden sind, einen Ring formen. Eine Stickstoffbase gemäß der vorliegenden Erfindung ist zu verstehen, als eine organische Verbindung mit zumindest einem Stickstoffatom mit einem freien Elektronenpaar. Eine Stickstoffbase kann also zumindest ein Proton aufnehmen, so dass dieses zumindest formal an das zumindest ein Stickstoffatom mit einem freien Elektronenpaar gebunden wird und bei Reaktion mit Wasser gemäß der folgenden Gleichgewichtsbeziehung basisch reagieren.

$$R_3N + H^+ \rightleftarrows R_3NH^+$$

$$R{=}N{-}R + H^+ \rightleftarrows R{=}NH^+{-}R$$

$$R_3N + H_2O \rightleftarrows R_3NH^+ + OH^-$$

$$R{=}N{-}R + H_2O \rightleftarrows R{=}NH^+{-}R + OH^-$$

Stickstoffbasen haben sich als besonders geeignet erwiesen, da sie im Gegensatz zu Hydroxiden und anderen Basen wie beispielsweise solchen Basen mit Carbonatanion, Hydrogencarbonatanion, Alkylcarbonatanion wie Methylcarbonat oder Ethylcarbonat, eine gute thermische Stabilität aufweisen und im Gegensatz zu Basen mit anorganischen Kationen eine gute Löslichkeit in ionischen Flüssigkeiten zeigen. Auch sind solche Basen, die mit Protonen zu Wasser oder Alkohol reagieren, ungünstig, da durch die zusätzliche Freisetzung von Wasser oder Alkoholen eine weitere Hydrolyse/Solvolyse des fluorhaltigen Anions unterstützt werden kann.

Weiters stehen Stickstoffbasen in einem $pK_S$-Wertbereich zur Verfügung, der für die erfindungsgemäßen Additive besonders geeignet ist.

In einer Ausführungsform weist das Additiv einen $pK_S$-Wert von höchstens 11,5 auf. Damit liegt der $pK_S$-Wert des Additivs bevorzugt im Bereich von 5 bis 11,5, mehr bevorzugt im Bereich von 6 bis 11, noch mehr bevorzugt 7 bis 10.

Ein weiterer Vorteil von Stickstoffbasen liegt in der Möglichkeit, dass diese gut derart ausgewählt sein können, dass sie ein Dealkylierungsprodukt des Kations $[A^+]$ darstellen. In einer bevorzugten Ausführungsform der Erfindung stellt nämlich das Additiv ein Dealkylierungsprodukt des Kations $[A^+]$ der ionischen Flüssigkeit dar.

Werden ionische Flüssigkeiten ganz allgemein für längere Zeit auf hohe Temperaturen (aber unterhalb ihres thermischen Zersetzungspunkts) erhitzt, wie z.B. typischerweise in der Anwendung als Wärmeträgerflüssigkeiten, kommt es in den meisten Fällen zu Transalkylierungsreaktionen, welche einer thermodynamisch getriebenen Equilibrierung der Alkylgruppen entsprechen. Verursacht werden solche Transalkylierungen bzw. Transarylierungen meist durch reversible nucleophile $SN_1$ oder $SN_2$ Substitutionen an den an Heteroatomen (N, P) gebundenen Seitenketten der Kationen

im Sinne einer reversen *Menschutkin*-Reaktion. Dabei greifen nucleophile Anionen wie z.B. Acetat, Dialkylphosphat oder Halogenid die Kationen unter Dealkylierung an, wobei in Folge aus den Kationen die entsprechenden nucleophilen tertiären Amine, tertiären Phosphine, N-Alkylimidazole, N-Alkylpyrrolidine, N-Alkylmorpholine, N-Alkylguanidine usw. als *Leaving-Group* entstehen sowie die durch Alkylierung aus den Anionen entstandenen elektrophilen Verbindungen, z.B. Alkylacetate, Trialkylphosphate und Alkylhalogenide. Das so entstandene Gemisch kann nun erneut im Sinne einer *Menschutkin*-Reaktion zurückreagieren, sodass in Folge Alkylgruppen ausgetauscht werden können. Je nach thermodynamischer Gleichgewichtslage und daher auch abhängig von der Temperatur, stellt sich nach einiger Zeit eine typische Verteilung der an den Heteroatomen der Kationen (N, P) gebundenen Alkylgruppen ein, sowie eine geringe Konzentration an neutralen, ungeladenen molekularen Species, welche durch Dealkylierung der Kationen und Alkylierung der Anionen entstanden sind und die Alkylgruppen quasi transportieren.

Auch bei ionischen Flüssigkeiten mit nicht-nucleophilen Anionen, wie z.B. $BF_4^-$ oder $PF_6^-$, können solche Transalkylierungsreaktionen auftreten, wobei in diesem Fall schon Spuren von den weiter oben schon beschrieben nucleophilen tertiären Aminen, tertiären Phosphinen, N-Alkylimidazolen, N-Alkylpyrrolidinen, N-Alkylmorpholinen, N-Alkylguanidinen usw. ausreichen können, welche z.B. Verunreinigungen wie nicht reagierte Edukte aus der Synthese der jeweiligen ionischen Flüssigkeit sein können. In diesem Fall greifen solche Nucleophile direkt die Kationen an, wobei sie selbst alkyliert werden und aus dem Kation durch Dealkylierung als *Leaving-Group* ein neues Nucleophil entsteht, das selber wieder angreifen kann.

Solche Transalkylierungsreaktionen sind für quaternäre Ammoniumsalze in der Literatur beschrieben ("Dynamic Covalent Chemistry of Nucleophilic Substitution, Component Exchange of Quaternary Ammonium Salts"; Kulchat, Sirinan; Lehn, Jean-Marie; Chem. Asian J. (2015), 10, 2484 - 2496). In dieser Literaturstelle werden analoge Reaktionen an quaternären Allyl- und Benzylammonium-Salzen und tertiären Aminen beschrieben und die Erstellung von ganzen "Libraries of Dynamic Ionic Liquids (DILs)" berichtet.

Von den Erfindern der vorliegenden Anmeldung wurde beobachtet, dass in der Anwendung von $BF_4^-$ bzw. $PF_6^-$ basierenden ionischen Flüssigkeiten insbesondere im Hochtemperaturbereich (z.B. Wärmeträgerflüssigkeiten) mit Zusatz von Additiven eine deutliche Transalkylierung stattfindet (Beispiel 3, Fig. 2-4). Das neu entstehende, thermisch equilibrierte Gemisch besitzt potentiell eine nahezu unkontrollierbare Vielzahl verschiedener Kationen (Fig. 4). Somit kann es zu einer komplexen Veränderung der Zusammensetzung der $BF_4^-$ bzw. $PF_6^-$ basierenden ionischen Flüssigkeit und möglicherweise zu veränderten und unerwünschten physikalischen und chemischen Eigenschaften kommen.

Es ist somit von Vorteil, wenn das Additiv derart ausgewählt ist, dass es formal aus dem Kation [$A^+$] der ionischen Flüssigkeit durch Dealkylierung abgeleitet werden können (im Sinne einer reversen *Menschutkin*-Reaktion), d.h. dass das Additiv ein Dealkylierungsprodukt des Kations [$A^+$] darstellt. Ein Dealkylierungsprodukt des Kations [$A^+$] ist also ein mögliches (Zwischen)produkt der Transalkylierung bzw. Transarylierung des Kations [$A^+$].:

$$[A^+] + [\text{Additiv}] \rightleftarrows [\text{Dealkylierungsprodukt}] + [\text{Alkyl-Additiv}^+]$$

Zum Beispiel ist bei einem Ammonium-Kation der Formel $[R^{1'}R^1R^2R^3N]^+$ ein Dealkylierungsprodukt des Ammonium-Kations ist, eines der Amine $R^1R^2R^3N$, $R^{1'}R^1R^2N$ oder $R^{1'}R^1R^3N$. Wenn das Additiv also ein solches Amin ist, entsteht bei der Transalkylierung ein [Alkyl-Additiv$^+$], das dem Ammonium-Kation entspricht.

Auch bevorzugt sind Additive, die zumindest der gleichen strukturellen Klasse angehört wie das Kation der ionischen Flüssigkeit und sich nur geringfügig, z.B. in der Länge der Alkylseitenketten, unterscheiden. Eine solches Beispiel ist in Figur 2 gezeigt, in der das Additiv 1-Ethylimidazol mit 1-Ethyl-3-methylimidazoliumtetrafluoroborat ein Gleichgewicht bildet, indem durch Transalkylierung wieder 1-Ethyl-3-methylimidazolium (Reaktionen a. und d.) entsteht ([Alkyl-Additiv$^+$] und [$A^+$] sind gleich) oder 1,3-Diethylimidazolium (Reaktion b., [Alkyl-Additiv$^+$] und [$A^+$] sind ähnlich) bzw. 1,3-Dimethylimidazolium (Reaktion c., [Alkyl-Additiv$^+$] und[$A^+$] sind ähnlich).

Die verwendete Base 1-Ethylimidazol und die entstehende Base 1-Methylimidazol zeichnen sich also dadurch aus, dass sie formal die Ausgangsstoffe der Synthese des 1,3-Dialkylimidazolium-Kations sind (Alkylierung in einer *Menschutkin*-Reaktion) bzw. formal aus dem Kation durch Dealkylierung abgeleitet werden können (reverse *Menschutkin*-Reaktion) und somit keine neue "Chemie" in das System einbringen.

Der Fachmann kann ein Additiv, das ein Dealkylierungsprodukt des Kations [$A^+$] der ionischen Flüssigkeit ist, ausgehend von der ionischen Flüssigkeit und ohne Aufwand auswählen. Auch die Bedingung, dass das Additiv einen pKs-Wert von zumindest 5 aufweist, kann der Fachmann basierend auf der Literatur oder einfachen Experimenten bestimmen (geeignete Quellen sind beispielsweise in den Beispielen angegeben).

Geeignete Kationen [$A^+$] der ionischen Flüssigkeit sind in einer Ausführungsform ausgewählt aus der Gruppe bestehend aus Ammonium, Phosphonium, Guanidinium, Pyridinium, Pyrimidinium, Pyridazinium, Pyrazinium, Piperidinium, Morpholinium, Piperazinium, Pyrrolium, Pyrrolidinium, Oxazolium, Thiazolium, Triazinium, Imidazolium, und Triazolium.

Weiters ist für die Kationen bevorzugt, wenn diese als "quaternäre" Verbindung vorliegt. "Quaternär" ist im Sinne der vorliegenden Anmeldung so zu verstehen, dass alle Valenzen eines Stickstoff- oder Phosphoratoms stabil

organisch gebunden sind, also kein freies, nicht bindendes Elektronenpaar mehr existiert. Dies trifft auf vierfach alkylierte Ammonium- oder Phosphoniumsalze ebenso zu, wie auf in Aromaten eingegliederte Stickstoffe (Heteroaromaten) mit einem dritten Substituenten, der nicht Wasserstoff ist, beispielsweise Pyridinium Salze. Entscheidend für die erfindungsgemäße Eignung ist dabei die Eigenschaft der konstanten positiven Ladung. Diese Eigenschaft weist auch protoniertes Guanidinium auf, da der Wasserstoff eine so geringe Azidität hat, dass auch hier eine konstante positive Ladung gewährleistet ist. Besonders bevorzugt sind daher - mit Ausnahme von Guanidinium - alle Reste an den formal ladungstragenden Heteroatomen (Stickstoff oder Phosphor) nicht Wasserstoff. Das Kation [$A^+$] der ionischen Flüssigkeit ist daher bevorzugt ausgewählt aus der Gruppe bestehend aus quaternären Ammonium, Phosphonium, Guanidinium, Pyridinium, Pyrimidinium, Pyridazinium, Pyrazinium, Piperidinium, Morpholinium, Piperazinium, Pyrrolium, Pyrrolidinium, Oxazolium, Thiazolium, Triazinium, Imidazolium, Triazolium, und protonierten Guanidinium.

Ganz allgemein sind Kationen und Additive mit abstrahierbaren Wasserstoffen weniger bevorzugt, da diese ebenfalls mit $BF_4^-$ und $PF_6^-$ reagieren können. Bevorzugt sind alle Wasserstoffatome des Additivs kovalent an Kohlenstoffatome gebunden. Ein solches Additiv wird als aprotisch beschrieben. Beispielsweise sind tertiäre Amine gegenüber sekundären oder primären Aminen bevorzugt. Das betrifft auch die mögliche Substitution, d.h. dass protische Funktionen, wie beispielsweise Substituenten mit Hydroxy-, Thiol- und primäre bzw. sekundäre Aminogruppen, nicht bevorzugt sind.

Bei Phosphonium-Kationen der Formel $[R^{1'}R^1R^2R^3P]^+$, sind Additive, die ein Dealkylierungsprodukt des Kations $[R^{1'}R^1R^2R^3P]^+$ darstellend die Phosphine $R^1R^2R^3P$, $R^{1'}R^1R^2P$ oder $R^{1'}R^1R^3P$. Phosphine sind als Additive weniger bevorzugt, da Trialkylphosphine nicht stabil genug sind, leicht mit Sauerstoff zu den entsprechenden Trialkylphosphinoxiden reagieren können und oftmals pyrophor sind.

Dealkylierungsprodukte der Kationen Ammonium, Guanidinium, Pyridinium, Pyrimidinium, Pyridazinium, Pyrazinium, Piperidinium, Morpholinium, Piperazinium, Pyrrolium, Pyrrolidinium, Oxazolium, Thiazolium, Triazinium, Imidazolium, bzw. Triazolium sind die bevorzugten Stickstoffbasen ausgewählt aus Amin, Guanidin, Pyridin, Pyrimidin, Pyridazin, Pyrazin, Piperidin, Morpholin, Piperazin, Pyrrol, Pyrrolidin, Oxazol, Thiazol, Triazin, Imidazol, bzw. Triazol.

In einer Ausführungsform steht $[A]^+$ für ein Ammonium-Kation $[R^{1'}R^1R^2R^3N]^+$, ein Phosphonium-Kation $[R^{1'}R^1R^2R^3P]^+$, ein heteroaromatisches Kation oder ein Guanidinium Kation $R^3R^{3'}N(C{=}NR^1R^{1'})NR^2R^{2'}$ der Formel

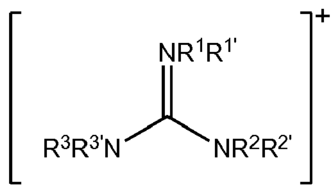

worin $R^1$, $R^{1'}$, $R^2$, $R^{2'}$ und $R^3$, $R^{3'}$ für organische Reste stehen und im Falle von Guanidinium für organische Reste oder Wasserstoff stehen, z.B. jeweils unabhängig voneinander für Wasserstoff (nur Guanidinium), unsubstituiertes Alkyl, Alkenyl, Alkinyl, Cycloalkyl, Cycloalkenyl, Aryl oder gesättigtes oder ungesättigtes Heterocyclyl, wie z.B. Heteroaryl, stehen, wobei die 7 letztgenannten Reste (Alkyl, Alkenyl, Alkinyl, Cycloalkyl, Cycloalkenyl, Aryl oder Heterocyclyl) jeweils unabhängig voneinander unsubstituiert oder substituiert sein können durch

ein oder mehr Halogen und/oder 1 bis 3 Reste ausgewählt aus

$C_1$-$C_6$-Alkyl, Aryl, gesättigtes oder ungesättigtes Heterocyclyl, wie Heteroaryl, $C_{3-7}$-Cycloalkyl, Halogen, $OR^c$, $NR^cR^d$, $COR^c$, $COOR^c$, $CO{-}NR^cR^d$, worin

$R^c$ und $R^d$ unabhängig voneinander für $C_1$-$C_6$-Alkyl, $C_1$-$C_6$-Halogenalkyl, Cycloalkyl, wie Cyclopentyl, Cyclohexyl, Phenyl, Tolyl oder Benzyl stehen; oder

zwei der Reste $R^1$, $R^{1'}$, $R^2$, $R^{2'}$, $R^3$, $R^{3'}$ bilden gemeinsam mit dem Heteroatom, an welchem sie gebunden sind, einen gesättigten oder ungesättigten Ring aus, der unsubstituiert oder substituiert ist, und wobei jede Kohlenstoffkette durch ein oder mehrere Heteroatome ausgewählt aus der Gruppe O, S oder $N(C_1$-$C_4$-Alkyl) unterbrochen sein kann.

Bevorzugt ist $[A]^+$ ein ungesättigter Ring und insbesondere ausgewählt aus einem 5- oder 6-gliedrigen Heteroaromat, der mindestens ein Stickstoffatom sowie gegebenenfalls ein Sauerstoff- oder Schwefelatom aufweist, und der unsubstituiert oder substituiert ist und/oder anelliert ist,

insbesondere ausgewählt aus der Gruppe der Formel

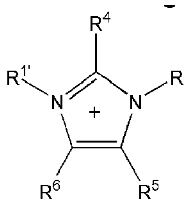 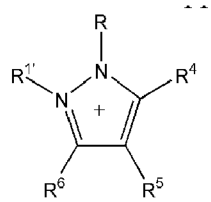 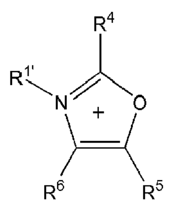 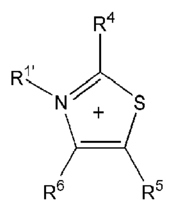 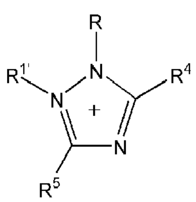

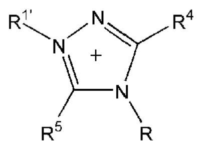
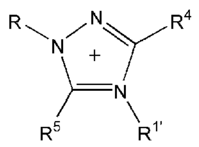
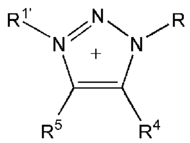
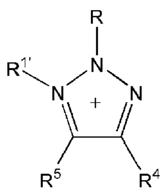
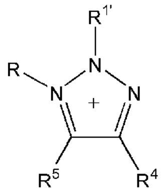

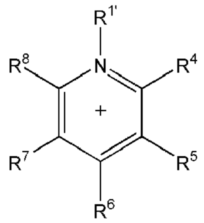
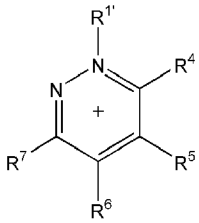
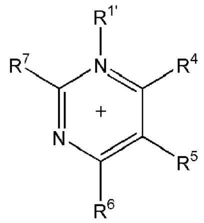
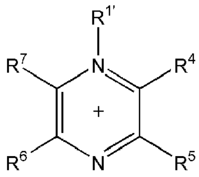

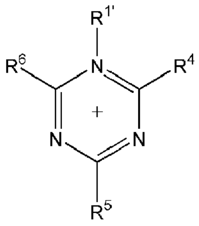
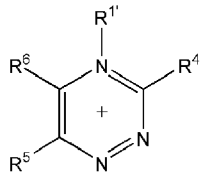
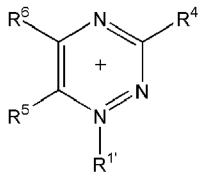
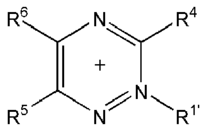

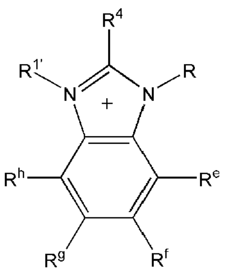
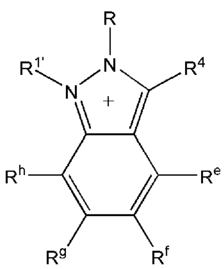
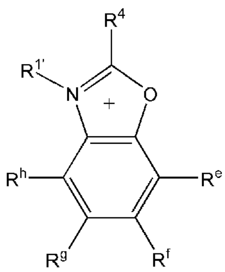
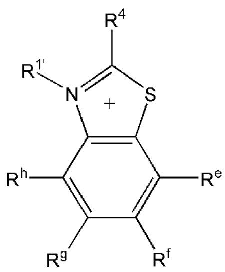

worin

R, $R^{1'}$ unabhängig voneinander $(C_1-C_{30})$Alkyl, $(C_3-C_{12})$Cycloalkyl, $(C_2-C_{30})$Alkenyl, $(C_3-C_{12})$Cycloalkenyl, Aryl oder gesättigtes oder ungesättigtes Heterocyclyl, insbesondere Heteroaryl, wobei die 6 letztgenannten Reste (d.h. $(C_1-C_{30})$Alkyl, $(C_3-C_{12})$Cycloalkyl, $(C_2-C_{30})$Alkenyl, $(C_3-C_{12})$Cycloalkenyl, Aryl oder Heterocyclyl) jeweils unabhängig voneinander unsubsutituiert sind, oder substitutiert durch

ein oder mehrere Halogenreste, und/oder 1 bis 3 Reste ausgewählt aus der Gruppe $(C_1-C_{30})$Alkyl, Aryl, gesättigtes oder ungesättigtes Heterocyclyl, insbesondere Heteroaryl, $(C_3-C_7)$Cycloalkyl, Halogen, $OR^c$, $NR^cR^d$, $COR^c$, $COOR^c$, $CO-NR^cR^d$, worin $R^c$ und $R^d$ unabhängig voneinander für $(C_1-C_6)$Alkyl, Halo$(C_1-C_6)$alkyl, Cycloalkyl, insbesondere Cyclopentyl, Cyclohexyl, Phenyl, Tolyl oder Benzyl stehen;

$R^4$, $R^5$, $R^6$, $R^7$, $R^8$ unabhängig voneinander Wasserstoff, $OR^c$, $NR^cR^d$, $COR^c$, $COOR^c$, $CO-NR^cR^d$, $(C_1-C_{30})$Alkyl, $(C_3-C_{12})$Cycloalkyl, $(C_2-C_{30})$Alkenyl, $(C_3-C_{12})$Cycloalkenyl, Aryl oder gesättigtes oder ungesättigtes Heterocyclyl, insbesondere Heteroaryl, bedeuten, wobei die 6 letztgenannten Reste jeweils unabhängig voneinander unsubstiuiert sind, oder substituiert durch

1 bis 3 Reste ausgewählt aus der Gruppe
(C1-C6)Alkyl, Aryl, gesättigtes oder ungesättigtes Heterocyclyl, insbesondere Heteroaryl, $(C_3-C_7)$Cycloalkyl, Halogen, $OR^c$, $SR^c$, $NR^cR^d$, $COR^c$, $COOR^c$, $CO-NR^cR^d$, wobei $R^c$ und $R^d$ unabhängig voneinander $(C_1-C_6)$Alkyl, Halo$(C_1-C_6)$alkyl, Cycloalkyl, insbesondere Cyclopentyl, Cyclohexyl, Phenyl, Tolyl oder Benzyl,

oder

zwei der Reste R, $R^4$, $R^5$, $R^6$, $R^7$, $R^8$, welche benachbart sind, bilden gemeinsam mit dem Atom, an welchem sie

gebunden sind, einen Ring aus, wobei dieser ungesättigt oder gesättigt, insbsondere aromatisch, unsubstituiert oder substituiert sein kann und wobei die durch die betreffenden Reste gebildete Kohlenstoffkette durch ein oder mehrere Heteroatome ausgewählt aus der Gruppe O, S, N, N($C_1$-$C_4$)Alkyl unterbrochen sein kann;
$R^e$, $R^f$, $R^g$, $R^h$ unabhängig voneinander Wasserstoff, Alkyl-, Alkenyl-, Alkinyl-, Cycloalkyl-, Cycloalkenyl-, Aryl- oder Heteroarylreste, bedeuten, wobei die 7 letztgenannten Reste jeweils unabhängig voneinander ein oder mehrere Halogenreste tragen können und/oder 1 bis 3 Reste ausgewählt aus der Gruppe ($C_1$-$C_6$)Alkyl, Aryl, Heteroaryl, ($C_3$-$C_7$)Cycloalkyl, Halogen, $OR^c$, $NR^cR^d$, $COR^c$, $COOR^c$, CO-$NR^cR^d$, wobei $R^c$ und $R^d$ unabhängig voneinander für ($C_1$-$C_6$)Alkyl, Halo($C_1$-$C_6$)alkyl, Cycloalkyl, insbesondere Cyclopentyl, Cyclohexyl, Phenyl, Tolyl oder Benzyl bedeuten.

Besonders bevorzugt ist dabei, wenn das Kation ausgewählt ist aus der Gruppe bestehend aus Guanidinium (protoniertes Guanidin); 1,1,3,3-Tetramethylguanidinium, 1,1,2,3,3-Pentamethylguanidinium, 1,1,2,2,3,3-Hexamethylguanidinium, Diethyldimethylammonium, Dipropyldimethylammonium, Dibutyldimethylammonium, Dihexyldimethyl-ammonium, Dioctyldimethylammonium, Triethylmethylammonium, Tripropylmethylammonium, Tributylmethylammonium, Trihexylmethylammonium, Trioctylmethylammonium, Trimethylethylammonium, Trimethylpropylammonium, Trimethylbutylammonium, Trimethylhexyl-ammonium, Trimethyloctylammonium, Tetramethylammonium, Tetraethylammonium, Tetrapropylammonium, Tetrabutylammonium, Tetrahexylammonium, Tetraoctylammonium, 2-Methoxyethyl-trimethylammonium (O-Methyl-Cholinium), 2-Methoxyethyl-diethylmethylammonium (DEME), Tris-(2-Methoxyethyl)-ammonium, Tris-(2-Methoxyethyl)-methylammonium, Tris-(2-Methoxyethyl)-ethylammonium, Bis-(2-Methoxyethyl)-dimethyl-ammonium, Triallylmethylammonium, Tetramethylphosphonium, Triethylmethylphosphonium, Tripropylmethylphosphonium, Tributylmethylphosphonium, Trihexylmethylphosphonium, Trioctylmethylphosphonium, Tetradecyl(trihexyl)phosphonium, Triisobutyl(methyl)phosphonium, Ethyl-(tributyl)-phosphonium, Octyl-(tributyl)-phosphonium, N-Decyl-N-methylpyrrolidinium, N-Octyl-N-methylpyrrolidinium, N-Hexyl-N-methylpyrrolidinium N-Butyl-N-methylpyrrolidinium, N-Propyl-N-methylpyrrolidinium, N-Ethyl-N-methylpyrrolidinium, N,N-dimethylpyrrolidinium, N-Allyl-N-methylpyrrolidinium, N-Decyl-N-methylmorpholinium, N-Octyl-N-methylmorpholinium, N-Hexyl-N-methylmorpholinium N-Butyl-N-methylmorpholinium, N-Propyl-N-methylmorpholinium, N-Ethyl-N-methylmorpholinium, N,N-Dimethylmorpholinium, N-Allyl-N-methylmorpholinium, N-Decyl-N-methylpiperidinium, N-Octyl-N-methylpiperidinium, N-Hexyl-N-methylpiperidinium N-Butyl-N-methylpiperidinium, N-Propyl-N-methylpiperidinium, N-Ethyl-N-methylpiperidinium, N,N-Eimethylpiperidinium, und N-Allyl-N-methylpiperidinium.

Besonders bevorzugt ist auch ein Kation ausgewählt aus der Gruppe bestehend aus 1,3-Dimethyl-imidazolium, 1,2,3-Trimethylimidazolium, 1-Ethyl-3-methyl-imidazolium, 1-Vinyl-3-methyl-imidazolium, 1-Vinyl-2,3-dimethyl-imidazolium, 1-Butyl-3-methylimidazolium, 1-Propyl-3-methylimidazolium, 1-iso-Propyl-3-methylimidazolium, 1-Allyl-3-methylimidazolium, 1-Propyl-2,3-dimethylimidazolium, 1-iso-Propyl-2,3-dimethylimidazolium, 1-Allyl-2,3-dimethylimidazolium, 1-Ethyl-2,3-dimethylimidazolium, 1-Butyl-2,3-dimethylimidazolium, 1-Hexyl-3-methylimidazolium, 1-Octyl-3-methylimidazolium, 1-Decyl-3-methylimidazolium, 1,3-Diethylimidazolium, und 1,3-Dibutylimidazolium.

Weiters bevorzugt sind die protonierten Formen der starken Basen 1,5-Diazabicyclo[4.3.0]non-5-en (DBN); 1,8-Diazabicyclo[5.4.0]undec-7-en (DBU); 1,4-Diazabicyclo-[2.2.2]-octan (DABCO®); 7-Methyl-1,5,7-triazabicyclo[4.4.0]dec-5-ene (MTBD); 1,8-Bis-(dimethylamino)-naphthalin (Proton Sponge®); N,N,N',N'-Tetramethylethylendiamin (TMEDA); 4,5-Bis-(dimethyl-amino)-fluoren; 1,8-Bis-(hexamethyltriaminophosphazenyl)naphthalin. Die protonierten Formen dieser Superbasen haben ähnlich wie Guanidium Kationen eine konstante positive Ladung.

In einer anwendbaren, wenn auch nicht bevorzugten Ausführungsform ist das Additiv ausgewählt aus der Gruppe umfassend Carbonate, einschließlich Hydrogencarbonate, Alkylcarbonate wie Methylcarbonat oder Ethylcarbonat, Phosphate und Hydrogenphosphate, sowie Hydroxide. Bei diesen als Salz vorliegenden Additiven ist das Kation bevorzugt das Kation [$A^+$] der ionischen Flüssigkeit oder ein Alkalimetall. Zum Beispiel kann das Additiv ausgewählt sein aus quaternären Ammonium- oder Phosphoniumhydroxiden.

Hinsichtlich der Additive sind bevorzugt Amine, d.h. Stickstoffbasen, insbesondere Amine der allgemeinen Formel

$$R^9R^{10}R^{11}N,$$

wobei $R^9$, $R^{10}$, $R^{11}$ jeweils voneinander unabhängig für geradliniges oder verzweigtes $C_1$-$C_6$-Alkyl oder $C_2$-$C_6$-Alkenyl sowie maximal zwei der drei Reste $R^9$, $R^{10}$, $R^{11}$ jeweils voneinander unabhängig für Aryl, gesättigtes oder ungesättigtes Heterocyclyl, wie Heteroaryl, $C_{5-6}$-Cycloalkyl, Phenyl, Tolyl oder Benzyl stehen; oder zwei der Reste $R^9$, $R^{10}$, $R^{11}$ bilden gemeinsam mit dem Heteroatom, an welchem sie gebunden sind, einen gesättigten oder ungesättigten Ring aus, der unsubstituiert oder substituiert ist, und wobei jede Kohlenstoffkette durch ein oder mehrere Heteroatome ausgewählt aus der Gruppe O, S oder N($C_1$-$C_4$-Alkyl) unterbrochen sein kann.

Auch bevorzugt sind Stickstoffbasen ausgewählt aus der Gruppe bestehend aus den Formeln

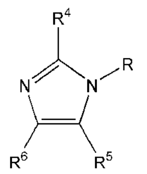 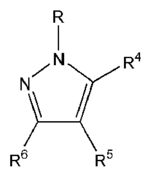 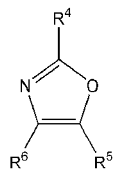 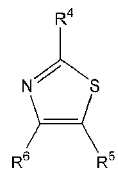 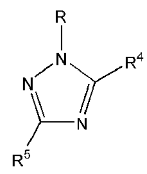

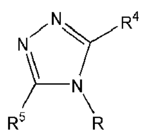 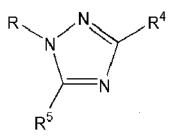 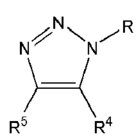 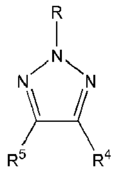

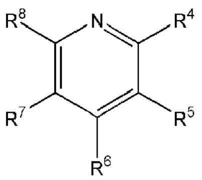 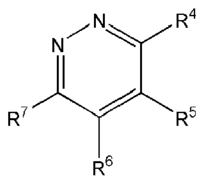 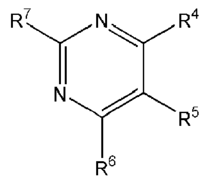 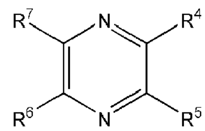

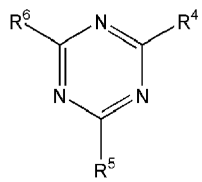 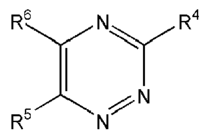 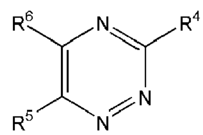 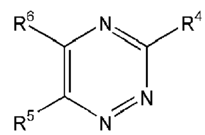

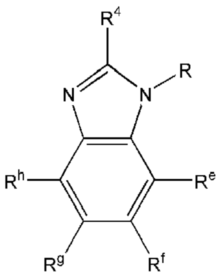 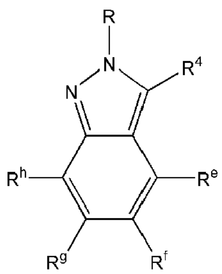 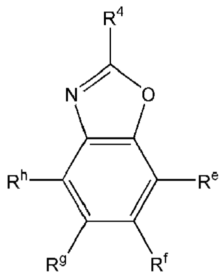 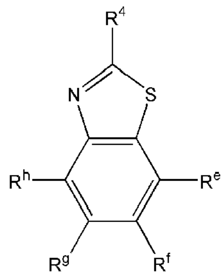

worin R, $R^4$, $R^5$, $R^6$, $R^7$, $R^8$, $R^e$, $R^f$, $R^g$, $R^h$ jeweils voneinander unabhängig für Wasserstoff, geradliniges oder verzweigtes $C_1$-$C_6$-Alkyl oder $C_2$-$C_6$-Alkenyl stehen sowie maximal zwei der vorgenannten Reste für Aryl, gesättigtes oder ungesättigtes Heterocyclyl, wie Heteroaryl, $C_{5}$-$_{6}$-Cycloalkyl, Phenyl, Tolyl oder Benzyl stehen; oder zwei der Reste R, $R^4$, $R^5$, $R^6$, $R^7$, $R^8$, $R^e$, $R^f$, $R^g$, $R^h$ bilden gemeinsam mit dem Heteroatom, an welchem sie gebunden sind, einen gesättigten oder ungesättigten Ring aus, der unsubstituiert oder substituiert ist, und wobei jede Kohlenstoffkette durch ein oder mehrere Heteroatome ausgewählt aus der Gruppe O, S oder N($C_1$-$C_4$-Alkyl) unterbrochen sein kann. Bevorzugt ist R nicht Wasserstoff.

Weiters sind geeignete Stickstoffbasen ausgewählt aus der Gruppe der starken Basen, bevorzugt aus der Gruppe bestehend aus 1,5-Diazabicyclo[4.3.0]non-5-en (DBN), 1,8-Diazabicyclo[5.4.0]undec-7-en (DBU), 1,4-Diazabicyclo-[2.2.2]-octan (DABCO®), 7-Methyl-1,5,7-triazabicyclo[4.4.0]dec-5-ene (MTBD), 1,8-Bis-(dimethylamino)-naphthalin (Proton Sponge®), N,N,N',N'-Tetramethylethylendiamin (TMEDA), 4,5-Bis-(dimethyl-amino)-fluoren, und 1,8-Bis-(hexamethyltriaminophosphazenyl)-naphthalin.

Besonders bevorzugte Stickstoffbasen sind neben jenen, welche sich formal durch Dealkylierung des Kations der jeweiligen ionischen Flüssigkeit ableiten lassen, solche die ausgewählt sind aus der Gruppe bestehend aus 1,1,3,3-Tetramethylguanidin, 1,1,2,3,3-Pentamethylguanidin, Diethylmethylamin, Dipropylmethylamin, Dibutylmethylamin, Dihexylmethylamin, Trimethylamin, Triethylamin, Tripropylamin, Triallylamin, Tributylamin, Trihexylamin, Dimethylethylamin, Dimethylpropylamin, Dimethylbutylamin, Dimethylhexylamin, 2-Methoxyethyl-dimethylamin, 2-Methoxyethyl-diethylamin, Bis-(2-Methoxyethyl)-methylamin, Bis-(2-Methoxyethyl)-ethylamin, Diallylmethylamin, Dimethylbenzylamin, Methyldibenzylamin, Tribenzylamin, N,N-Diisopropylethylamin, Pyrrolidin, N-Hexylpyrrolidin N-Butylpyrrolidin, N-Propylpyrrolidin, N-Ethylpyrrolidin, N-Methylpyrrolidin, N-Allylpyrrolidin, N-Benzylpyrrolidin, N-Hexylmorpholin N-Butylmorpholin, N-Propylmorpholin, N-Ethylmorpholin, N-Methylmorpholin, N-Allylmorpholin, N-Benzylmorpholin, Morpholin, N-Hexylpiperidin, N-Butyl-piperidin, N-Propylpiperidin, N-Ethylpiperidin, N-Methylpiperidin, N-Allylpiperidin, Piperazin, $C_1$-$C_6$ Dialkylpiperazin, 1H-Imidazol, 1-Methylimidazol, 1,2-Dimethylimidazol, 1-Ethylimidazol, 1-Ethyl-2-methylimidazol, 1-Butylimidazol, 1-Butyl-2-methylimidazol, 1-Propylimidazol, 1-iso-Propylimidazol, 1-Allylimidazol, 1-Hexylimidazol, 1-Octylimidazol, 1-Benzylimidazol, 1-Benzyl-2-methylimidazol, Pyridin, 4-(Dimethylamino)-pyridin, Benzotriazol, 1-Methylbenzotriazol, und 2-Mercaptobenzothiazol.

Weitere Bevorzugungen hinsichtlich des Additivs können in Zusammenhang mit der angestrebten Anwendung stehen. Beispielsweise kann bevorzugt sein, dass das Additiv (ebenso wie die meisten ionischen Flüssigkeiten) einen geringen Dampfdruck aufweist. Der Dampfdruck korreliert typischerweise mit dem Reinstoff-Siedepunkt des Additivs, wobei in der Zusammensetzung durch Wechselwirkung mit der ionischen Flüssigkeit niedrigere Dampfdrücke beobachtet werden (Henry'sches Gesetz). Dennoch kann ein möglichst hoher Siedepunkt und/oder eine hohe thermische Stabilität für das Additiv erwünscht sein. Auch diesbezüglich zeigen sich Stickstoffbasen, insbesondere aromatische Stickstoffbasen und sogenannte Superbasen, als geeignet.

Weitere bevorzugte Eigenschaften können sein, dass die Additive den Schmelzpunkt der Zusammensetzung senken (d.h. das Additiv ein Mittel zur Gefrierpunktserniedrigung ist und/oder die Zusammensetzung ein Eutektikum darstellt) oder die Viskosität der Zusammensetzung senken, oder als Korrosionsschutz, Lösungsvermittler oder Antioxidans wirken.

In einer Ausführungsform liegt in der Zusammensetzung das Additiv in einen Anteil von höchstens 20 mol%, bevorzugt von höchstens 10 mol%, oder von höchstens 5 mol%, vor. Dabei steht mol% für den Stoffmengenanteil des Additivs bezogen auf die Summe der Stoffmengen in der Zusammensetzung steht bzw. bevorzugt auf den Stoffmengenanteil des Additivs bezogen auf die Summe der Stoffmengen von ionischer Flüssigkeit $[A^+]$ $[B^-]$ und Additiv. Besonders bevorzugt liegt das Additiv in einen Anteil von ungefähr 5 mol% bis ungefähr 10 mol% vor. In einer Ausführungsform liegt das Additiv in einen Anteil von zumindest 1 mol% vor.

Auch bevorzugt liegt das Additiv bezogen auf die Zusammensetzung in einen Gewichtsanteil von höchstens 20 %, bevorzugt von höchstens 10 %, besonders bevorzugt von höchstens 5 %, vor.

Der Zusammensetzung gemäß vorliegender Erfindung können weiters Metallsalze $[M]^{a+}[BF_4]^-_a$ und/oder $[M]^{b+}[PF_6]^-_b$ zugesetzt sein, wobei a und b voneinander unabhängig die Zahlen 1, 2, 3 oder 4 bedeuten. Bevorzugte Metallkationen $[M]^{a+}$ oder $[M]^{b+}$ sind dabei $Cr^{+2}$, $Cr^{+3}$, $Co^{+2}$, $Co^{+3}$, $Cu^{+1}$, $Cu^{+2}$, $Fe^{+2}$, $Fe^{+3}$, $Mn^{+2}$, $Mn^{+3}$, $Ni^{+2}$, $Ni^{+3}$, $Ti^{+2}$, $Ti^{+3}$, $Li^+$, $Na^+$, $K^+$, $Cs^+$, $Mg^{2+}$, $Ca^{2+}$, $Ba^2$, $Sr^{2+}$, $Zr^{4+}$, $Sn^{2+}$, $Sn^{4+}$, $Ag^+$, $Zn^{2+}$ und $Al^{3+}$, besonders bevorzugte sind $Co^{+2}$, $Co^{+3}$, $Cu^{+1}$, $Cu^{+2}$, $Fe^{+2}$, $Fe^{+3}$, $Mn^{+2}$, $Mn^{+3}$, $Ti^{+2}$, $Ti^{+3}$, $Li^+$, $Na^+$, $K^+$, $Mg^{2+}$, $Ca^{2+}$, $Zn^{2+}$ und $Al^{3+}$. Bevorzugt liegen Metallsalze in einen Gewichtsanteil von 5 bis 50 m% bezogen auf die Zusammensetzung vor.

In einer weiteren bevorzugten Ausführungsform ist das Anion $[B^-]$ der ionischen Flüssigkeit $BF_4^-$. Zusammensetzungen mit dem Anion $BF_4^-$ zeigen eine vergleichsweise stärkere Erniedrigung des pH-Werts, wenn sie in Kontakt mit Wasser kommen, als Zusammensetzungen mit dem Anion $PF_6^-$ (Vergleich Beispiel 1 und 2).

In einem weiteren Aspekt betrifft die Erfindung eine Verwendung der erfindungsgemäßen Zusammensetzung als Elektrolyt, Wärmeträger, Kühlmittel, Kraftübertragungsmedium (Hydraulikmedium), Schmiermittel, Dichtungsmittel, Lösungsmittel, Medium zum thermischen Quenchen von Metallen (Härtung, Oberflächenbehandlung), Antistatikum, Flammschutzmittel und/oder Weichmacher (z.B. für Kunststoffzusammensetzungen).

Für diese Anwendungen sind die erfindungsgemäßen Zusammensetzungen besonders gut geeignet, da sie die Vorteile der fluorierten ionischen Flüssigkeiten aufweisen und dennoch ein verringertes Risiko einer Freisetzung von Fluorwasserstoff aufweisen, wenn die Zusammensetzung mit Wasser und/oder Alkohol(en) in Kontakt kommt.

## DETAILLIERTE BESCHREIBUNG DER ERFINDUNG

Im Folgenden werden Experimente gezeigt, die erfindungsgemäßen Zusammensetzungen veranschaulichen und/oder für das Verständnis der Erfindung hilfreich sind. Es versteht sich, das die Ausführungsvarianten beispielhaft sind.

Die Figuren, die dabei zur Erklärung dienen, zeigen Folgendes:

Fig. 1 zeigt die chronologische Entwicklung der Anzahl der Publikationen mit $BF_4^-$ (durchgezogen) und $PF_6^-$ (strichliert). Die zahlenmäßige Entwicklung für alle Publikationen ("Literatur") sind mit dicken Linien und die der darin enthaltene Anzahl an Patentliteratur ("Patente") mit dünnen Linien dargestellt. Die Zahlen basieren auf Treffern einer Recherche mit Hilfe der CAS SciFinder™ Datenbank und den Suchbegriffen "tetrafluoroborat ionic liquid" und "hexaphosphoborat ionic liquid", wobei die Suche nicht auf Treffer mit den wortwörtlichen Suchbegriffen eingeschränkt wurde (implementiert als "concept search").

Fig. 2 zeigt mögliche Transalkylierungsreaktionen a.-d. von EMIM-$BF_4$ mit Ethylimidazol, die zu der Bildung einer "Library of Dynamic Ionic Liquids" führen, bestehend aus EMIM-$BF_4$, DMIM-$BF_4$, DEIM-$BF_4$ und den Basen 1-Ethylimidazol und 1-Methylimidazol, welche alle miteinander im thermodynamischen Gleichgewicht stehen, wie in

Beispiel 3.1 beschrieben.

Fig. 3 zeigt möglicher Transalkylierungsreaktionen von EMIM-$BF_4$ mit Butylimidazol und entstehende Transalkylierungsprodukte (A) sowie ein Chromatogramm der detektierten Produkte (B), mit dem die Bildung von 1-Ethylimidazol (1 - EI), 1-Ethyl-3-methylimidazolium (2 - EMIM) und 1-Butyl-3-methylimidazolium (3 - BMIM) gezeigt wird, wie in Beispiel 3.2 beschrieben.

Fig. 4 zeigt mögliche Transalkylierungsreaktionen EMIM-BF4 mit 1-Butylpyrrolidin, die zu der Bildung einer "Library of Dynamic Ionic Liquids" führen, bestehend aus einer Vielzahl verschiedener Kationen und Stickstoffbasen, wie in Beispiel 3.3 beschrieben.

Fig. 5 zeigt das Cyclovoltammogramm von trockenem EMIM-BF4.

Fig. 6 zeigt das Cyclovoltammogramm von EMIM-BF4 mit 1 m% des Additivs DBU.

BEISPIELE

**Allgemeine Messmethoden und Verwendete Materialien**

pH-Werte der Mischungen aus Wasser und $BF_4^-$ bzw. $PF_6^-$ basierenden ionischen Flüssigkeiten wurden mit einem handelsüblichen, sorgfältig kalibrierten Labor-pH-Meter mit Standard-Glaselektrode bei Raumtemperatur gemessen, wobei Konzentration von ≤ 80% ionischer Flüssigkeit bzw. ≥ 20% Wasser in der Literatur als mit einer Glaselektrode messbar evaluiert worden sind (siehe z.B. Bou Malhama, I.; Letelliera, P.; Turmine, M.; Talanta 77 (2008) 48-52). Der pH50-Werte ist der pH-Wert einer frisch hergestellten und sofort vermessenen 50%igen wässrigen Lösung (m/m). Im Falle von wasserunmischbaren ionischen Flüssigkeiten wurde nach Schütteln und Phasentrennung die wässrige Phase vermessen. Ggf. wurde dabei bis über den Schmelzpunkt der ionischen Flüssigkeit erwärmt.

Brechungsindizes wurden mit einem bei 20 ± 0,1 °C thermostatisierten Abbe-Refraktometer gemessen (soweit nicht anders angegeben). Bei nicht wassermischbaren Proben wurde jeweils die organische Phase vermessen.

Hochdruckflüssigkeitschromatographie-Messungen (HPLC) wurden wie folgt durchgeführt: Proben wurden in HPLC Eluent (60 m% Reinstwasser, 40 m% Acetonitril und 0,22 m% Ameisensäure) auf eine Konzentration von 0,1 mg/mL verdünnt und unverzüglich auf einem Dionex Ultimate 3000 System mit einem Ultimate 3000 RS UV-Detektor (210 nm) vermessen. Als analytische Trennsäule wurde eine Umkehrphase mit schwach sauren Ionentauschergruppen eingesetzt (SIELC Primesep 200, 3,2 x 250 mm, Partikelgröße 5 μm) und als mobile Phase diente der bereits beschriebene Eluent. Die Messung erfolgt isokratisch über eine Dauer von 25 min bei einem Fluss von 0,5 ml/min, einer Temperatur im Säulenofen von 25°C und mit einem Injektionsvolumen von 20 μL. Die Identifikation und Quantifizierung der Peaks erfolgte durch Kalibration mit entsprechenden hochreinen Standardsubstanzen und basiert auf jahrelangen Erfahrungswerten. Die Integration der Chromatogramme erfolgt manuell.

Ionenchromatographie-Messungen (IC) wurden wie folgt durchgeführt: Proben wurden mit dem IC Eluenten (90 m% Reinstwasser, 10 m% Acetonitril) im linearen Bereich der Kalibration auf 0,8 - 15 mg/mL verdünnt und auf einem Dionex ICS-5000 System mit einem Dionex ICS-3000 Leitfähigkeitsdetektor in Kombination mit einem Thermo Scientific AMMS 300 Mikromembransuppressor (regeneriert durch 50 mM Schwefelsäure, Flussrate 0,4 mL/min) vermessen. Als stationäre Phase dient eine Ionentauschersäule (Thermo Scientific Dionex IonPac AS20 RFIC, 4 x 250 mm, Partikelgröße 7,5 μm) und die mobile Phase variierte nach folgendem 42,5 minütigem Programm: 1.) -7,5 min - 0 min 5% 100 mM NaOH, 20% Acetonitril (isokratisch) / 2.) 0 min - 15 min 10-60% 100 mM NaOH (Gradient), 20% Acetonitril/ 3.) 15 min - 35 min 10-60% 100 mM NaOH (Gradient), 20% Acetonitril: Die Flussrate ist konstant bei 1 mL/min und der Säulenofen auf 35 °C. Die Identifikation und Quantifizierung der Peaks erfolgte durch Kalibration mit entsprechenden hochreinen Standardsubstanzen und basiert auf jahrelangen Erfahrungswerten. Die Integration der Chromatogramme erfolgt manuell. Ionenchromatographie wurde verwendet um beispielsweise den Gehalt Fluorid zu bestimmen.

Karl Fischer Titration (KFT) zur Bestimmung von Wasseranteilen wurden mit einem Metrohm 890-Titrando System mit Tiamo™ Software durchgeführt.

Chemikalien wurden im einschlägigen Chemikalienhandel mit einer Reinheit ≥ 98 m% bezogen und ohne weitere Aufreinigung verwendet. Ionische Flüssigkeiten wurden, sofern nicht anders beschrieben, nach unserem CBILS®-Verfahren in einer qualitätsgesicherten Reinheit ≥ 98 m% hergestellt (siehe Kalb, R.S. et al., Phys. Chem. Chem. Phys. (2016), 18, 31904; Kalb, R.S. et al., React. Chem. Eng. (2017), 2, 432). 1-Ethyl-3-methylimidazolium methylcarbonat, Tributylmethylammonium methylcarbonat und Tributylmethylphosphonium methylcarbonat wurden ebenfalls über das CBILS®-Verfahren als 30 - 50%ige methanolische Lösungen hergestellt (Intermediate zur Herstellung von ionischen Flüssigkeiten, stabilisiert in Methanol) und als solche zu den ionischen Flüssigkeiten zudosiert; das Lösungsmittel wurde jeweils unmittelbar *in vacuo* entfernt.

**Beispiel 1 - Untersuchung von verschiedenen Additiven in Zusammensetzungen mit der ionischen Flüssigkeit 1-Ethyl-3-methylimidazolium-tetrafluoroborat**

Eine Reihe verschiedener Basen wurden als Additive für die ionische Flüssigkeit 1-Ethyl-3-methylimidazolium-tetrafluoroborat (EMIM-BF4, CAS 143314-16-3, $M_r$ = 197,97 g/mol) untersucht.

Kommerziell erhältliches EMIM-BF4 wurde in einem druckfest verschließbaren Teflongefäß ($p_{max}$ = 5 bar) jeweils mit 10 mol% der zu untersuchenden Base versetzt und das Gemisch mit Reinstwasser auf exakt 50 m% verdünnt; diese verhältnismäßig große Konzentration an Wasser wurde ausgewählt, um Hydrolyseprozesse deutlich beobachten zu können.

Nach Zugabe eines Magnetrührstabes aus Teflon wurde die nun 50%ige wässrige Lösung homogenisiert. Es wurde sofort eine Probe entnommen (t = 0 Stunden), Aspekt und Farbe visuell bestimmt, der pH-Wert und der Brechungsindex gemessen, sowie eine HPLC und IC Analyse durchgeführt. Das Teflongefäß wurde nun druckdicht verschlossen und bei 80 ± 0,1 °C thermostatisiert und magnetisch gerührt. In regelmäßigen Abständen wurde das Gefäß nun rasch auf Raumtemperatur abgekühlt, der pH-Wert gemessen, wieder verschlossen und weiter bei 80 °C gerührt. Dies wurde so lange fortgeführt, bis der pH-Wert konstant blieb, was in allen Fällen nach spätestens 164 Stunden erreicht wurde. Am Ende der Messsequenz wurde nun wieder Aspekt und Farbe visuell bestimmt, der Brechungsindex gemessen, sowie eine HPLC und IC Analyse durchgeführt.

Als Referenzprobe wurde EMIM-BF4 ohne Zusatz eines basischen Additives auf 50 %m mit Reinstwasser verdünnt und analog behandelt, wobei bereits zu Beginn ein pH von 2,5 gemessen wurde (Probe "kein Additiv (unbehandelt)"). Ein Teil dieser Referenzprobe wurde mittels Ionenaustauscher in der Form der nicht ionischen, freien $NH_2$-Base (DOWEX® MWA-1) entsäuert, wobei Fluorwasserstoff vollständig entfernt wurde und sich ein pH von 7,1 zeigte (Probe "kein Additiv (mit Ionenaustauscher)".

**Fehler! Verweisquelle konnte nicht gefunden werden.** und 2 zeigen die Ergebnisse dieser Versuchsreihe geordnet nach absteigendem, konstantem pH-Wert (spätestens nach 164 Stunden Versuchsdauer), wobei Tabelle 1 insbesondere die relevanten Daten und Ergebnisse zum pH-Wert zusammenfasst und Tabelle 2 weitere Beobachtungen.

Tabelle 1:

| Nr. | Substanz | CAS | pKs in H2O | Quel le pKs | pH Start (0h) | pH Ende (164h) | ΔpH | %HF |
|---|---|---|---|---|---|---|---|---|
| 1 | TMEDA | 110-18-9 | 8,97 | 2 | 10,4 | 5,9 | 4,5 | 0,2 |
| 2 | N,N,N',N'-Tetramethylguanidine | 80-70-6 | 13,60 | 6 | 13,5 | 5,7 | 7,7 | 0,3 |
| 3 | Na2CO3 | 497-19-8 | 10,40 | 2 | 10,7 | 5,6 | 5,1 | 0,3 |
| 4 | 1-Ethylimidazol | 7098-07-9 | 7,26 | 1 | 8,2 | 5,5 | 2,7 | 0,5 |
| 5 | 1-Butylimidazol | 4316-42-1 | 7,21 | 1 | 8,5 | 5,4 | 3,1 | 0,5 |
| 6 | K2CO3 | 584-08-7 | 10,40 | 2 | 11,7 | 5,3 | 6,4 | 0,8 |
| 7 | 1-Methylimidazol | 616-47-7 | 7,21 | 1 | 8,3 | 5,3 | 3,0 | 0,8 |
| 8 | 2,6-Lutidine | 108-48-5 | 6,60 | 2 | 7,6 | 5,2 | 2,5 | 1,0 |
| 9 | 1-Ethyl-3-methylimidazolium methylcarbonat | 251102-25-7 | 6,50 | 8 | 8,2 | 5,1 | 3,2 | 1,3 |
| 10 | Natrium ethanolat | 141-52-6 | 14,00 | 9 | 13,2 | 5,0 | 8,2 | 1,4 |
| 11 | Tributylmethylammonium methylcarbonat | 274257-37-3 | 6,50 | 8 | 8,1 | 5,0 | 3,1 | 1,4 |
| 12 | Imidazol | 288-32-4 | 6,96 | 1 | 8,0 | 4,9 | 3,1 | 1,7 |
| 13 | tert. - Butylamin | 75-64-9 | 10,68 | 5 | 11,6 | 4,9 | 6,7 | 1,7 |
| 14 | KOH | 1310-58-3 | 14,00 | 9 | 13,6 | 4,9 | 8,6 | 1,7 |
| 15 | Tributylmethylphosphonium methylcarbonat | 120256-45-3 | 6,50 | 8 | 8,3 | 4,9 | 3,4 | 1,8 |
| 16 | DBU | 6674-22-2 | 13,50 | 6 | 13,8 | 4,9 | 8,9 | 1,8 |

(fortgesetzt)

| Nr. | Substanz | CAS | pKs in H2O | Quelle pKs | pH Start (0h) | pH Ende (164h) | ∆pH | %HF |
|---|---|---|---|---|---|---|---|---|
| 17 | Kalium tert.butoxid | 865-47-4 | 14,00 | 9 | 13,5 | 4,9 | 8,6 | 1,8 |
| 18 | L-Histidine | 71-00-1 | 6,00 | 2 | 6,6 | 4,9 | 1,7 | 1,8 |
| 19 | 1-Butylpyrrolidin | 767-10-2 | 10,60 | 7 | 11,5 | 4,9 | 6,6 | 1,8 |
| 20 | Dibutylamin | 111-92-2 | 11,07 | 4 | 11,7 | 4,9 | 6,8 | 1,9 |
| 21 | n-Butylamin | 109-73-9 | 10,67 | 4 | 11,5 | 4,9 | 6,6 | 1,9 |
| 22 | Triethylamin | 121-44-8 | 10,74 | 4 | 11,7 | 4,8 | 6,9 | 2,1 |
| 23 | Guanidinium-Carbonat | 593-85-1 | 9,83 | 3 | 10,7 | 4,8 | 5,9 | 2,1 |
| 24 | DBN | 3001-72-7 | 13,42 | 7 | 13,7 | 4,8 | 8,8 | 2,1 |
| 25 | sec.-Butylamin | 13952-84-6 | 10,56 | 5 | 11,6 | 4,8 | 6,8 | 2,4 |
| 26 | Triethanolamine | 102-71-6 | 7,74 | 2 | 9,1 | 4,8 | 4,3 | 2,4 |
| 27 | 1-Methylpyrrolidine | 120-94-5 | 10,32 | 5 | 11,4 | 4,8 | 6,6 | 2,5 |
| 28 | 1,2-Dimethylimidazol | 1739-84-0 | 8,21 | 1 | 9,1 | 4,7 | 4,3 | 2,6 |
| 29 | NaOH | 1310-73-2 | 14,00 | 9 | 13,0 | 4,7 | 8,3 | 2,6 |
| 30 | L-Arginin | 74-79-3 | 12,10 | 2 | 10,1 | 4,6 | 5,4 | 3,3 |
| 31 | Pyrrolidine | 123-75-1 | 11,27 | 5 | 12,3 | 4,6 | 7,6 | 3,4 |
| 32 | Tris-Base | 77-86-1 | 8,07 | 2 | 9,1 | 4,6 | 4,5 | 3,8 |
| 33 | Tributylamin | 102-82-9 | 9,05 | 4 | 9,3 | 4,5 | 4,8 | 4,3 |
| 34 | Pyridin | 110-86-1 | 5,23 | 2 | 6,2 | 4,5 | 1,8 | 5,0 |
| 35 | Trioctylamin | 1116-76-3 | 7,18 | 3 | 6,8 | 4,4 | 2,4 | 5,4 |
| 36 | Tetrabutylammonium hydroxid | 2052-49-5 | 14,00 | 9 | 13,4 | 4,4 | 9,0 | 5,6 |
| 37 | kein Additiv (mit Ionenaustauscher) | - | - | - | 7,1 | 3,9 | 3,2 | 15,7 |
| 38 | LiOH | 1310-65-2 | 14,00 | 9 | 12,2 | 3,7 | 8,4 | 21,1 |
| 39 | 2,6-Di-tert-butylpyridine | 585-48-8 | 3,58 | 2 | 3,8 | 2,6 | 1,1 | 77,8 |
| 40 | kein Additiv (unbehandelt) | - | - | - | 2,5 | 2,3 | 0,2 | 87,3 |

Tabelle 2:

| Nr. | Substanz | Sol. | $\Delta n_D^{20}$ | Änderung Aspekt & Farbe | Änderung HPLC, IC |
|---|---|---|---|---|---|
| 1 | TMEDA | + | 0,0039 | klar, von farblos nach leicht gelb | unverändert |
| 2 | N,N,N',N'-Tetramethylguanidine | + | 0,0010 | klar, farblos, unverändert | unverändert |
| 3 | Na2CO3 | ± | 0,0016 | klar, farblos, unverändert | unverändert |
| 4 | 1-Ethylimidazol | + | 0,0010 | klar, farblos, unverändert | unverändert |
| 5 | 1-Butylimidazol | + | 0,0020 | klar, farblos, unverändert | unverändert |
| 6 | K2CO3 | ± | 0,0013 | klar, farblos, unverändert | unverändert |
| 7 | 1-Methylimidazol | + | 0,0006 | klar, farblos, unverändert | unverändert |

(fortgesetzt)

| Nr. | Substanz | Sol. | $\Delta n_D^{20}$ | Änderung Aspekt & Farbe | Änderung HPLC, IC |
|---|---|---|---|---|---|
| 8 | 2,6-Lutidine | + | 0,0017 | klar, von farblos nach leicht gelb | unverändert |
| 9 | 1-Ethyl-3-methylimidazolium methylcarbonat | + | -0,0004 | klar, von farblos nach leicht gelb | unverändert |
| 10 | Natrium ethanolat | (±) | 0,0000 | klar, von farblos nach gelb | unverändert |
| 11 | Tributylmethylammonium methylcarbonat | + | -0,0004 | klar, farblos, unverändert | unverändert |
| 12 | Imidazol | + | 0,0005 | klar, farblos, unverändert | unverändert |
| 13 | tert.-Butylamin | + | 0,0013 | klar, farblos, unverändert | unverändert |
| 14 | KOH | (±) | -0,0004 | Niederschlag, leicht gelb, unverändert | unverändert |
| 15 | Tributylmethylphosphonium methylcarbonat | + | -0,0005 | klar, farblos, unverändert | unverändert |
| 16 | DBU | + | 0,0010 | klar, von farblos nach leicht gelb | unverändert |
| 17 | Kalium tert.butoxid | (±) | 0,0009 | Niederschlag, von gelb nach braun | unverändert |
| 18 | L-Histidine | ± | 0,0070 | Niederschlag, von farblos nach gelb | unverändert |
| 19 | 1-Butylpyrrolidin | + | 0,0030 | klar, farblos, unverändert | unverändert |
| 20 | Dibutylamin | + | 0,0025 | klar, farblos, unverändert | unverändert |
| 21 | n-Butylamin | + | 0,0020 | klar, farblos, unverändert | unverändert |
| 22 | Triethylamin | + | 0,0014 | klar, von farblos nach leicht gelb | unverändert |
| 23 | Guanidinium-Carbonat | ± | 0,0012 | klar, farblos, unverändert | unverändert |
| 24 | DBN | + | 0,0015 | klar, von farblos nach leicht gelb | unverändert |
| 25 | sec.-Butylamin | + | 0,0016 | klar, farblos, unverändert | unverändert |
| 26 | Triethanolamine | + | 0,0026 | klar, von farblos nach leicht gelb | unverändert |
| 27 | 1-Methylpyrrolidine | + | 0,0028 | klar, von farblos nach gelb | unverändert |
| 28 | 1,2-Dimethylimidazol | + | 0,0010 | klar, leicht gelb, unverändert | unverändert |
| 29 | NaOH | (±) | -0,0009 | klar, von farblos nach leicht gelb | unverändert |
| 30 | L-Arginin | ± | 0,0034 | klar, farblos, unverändert | unverändert |
| 31 | Pyrrolidine | + | 0,0020 | klar, von farblos nach leicht gelb | unverändert |
| 32 | Tris-Base | ± | 0,0025 | klar, farblos, unverändert | unverändert |
| 33 | Tributylamin | + | 0,0047 | klar, farblos, unverändert | unverändert |
| 34 | Pyridin | + | 0,0001 | klar, farblos, unverändert | unverändert |
| 35 | Trioctylamin | - | 0,0015 | trübe, von farblos nach gelb | unverändert |

(fortgesetzt)

| Nr. | Substanz | Sol. | $\Delta n_D^{20}$ | Änderung Aspekt & Farbe | Änderung HPLC, IC |
|---|---|---|---|---|---|
| 36 | Tetrabutylammonium hydroxid | + | -0,0006 | klar, farblos, unverändert | unverändert |
| 37 | kein Additiv (mit Ionenaustauscher) | | -0,0003 | klar, farblos, unverändert | unverändert |
| 38 | LiOH | (±) | 0,0005 | klar, farblos, unverändert | unverändert |
| 39 | 2,6-Di-tert-butylpyridine | - | 0,0000 | trübe, von farblos nach gelb | unverändert |
| 40 | kein Additiv (unbehandelt) | | 0,0008 | klar, farblos, unverändert | unverändert |

Die in Tabelle 1 und 2 verwendeten Abkürzungen und Beschriftungen sind wie folgt zu verstehen:

CAS Chemical Abstracts Nummer (**CAS**); Säurekonstante (**p*Ks* in $H_2O$**); Literaturquelle für $pK_S$-Werte (**Quelle pKs**); Löslichkeit von 10mol% in EMIM-BF4 ohne Zusatz von Wasser nach 24h Rühren bei 80 °C (visuelle Beobachtung): + vollständig, ± teilweise, - unlöslich, (±) teilweise unter starker Farbänderung und Trübung = Zersetzung (**Sol.**); pH-Differenz $pH_{Start}$-$pH_{Ende}$ (**ΔpH**); Differenz Brechungsindex $n_D^{20}$Ende - $n_D^{20}$start (**$\Delta n_D^{20}$**); Prozentsatz des bei konstantem $pH_{Ende}$ als Fluorwasserstoff (HF) vorliegenden Fluorids, berechnet aus der Henderson-Hasselbalch Gleichung und dem $pK_S$-Wert von 3,17 für HF (**% HF**); Änderung des Aspekts und der Farbe der wässrigen Reaktionsmischungen (**Änderung Aspekt & Farbe**)

Als Literaturquellen für pKs-Werte stehen in der Spalte "Quelle pKs" in Tabelle 1: **1** J. Heterocyclic Chem., 39, 287 (2002); **2** Wikipedia (deutsche und englische Websites); **3** Experimentelle Bestimmung durch Halbtitration mit 1N HCl in Methanol mittels Metrohm Solvotrode (Nr. 6.0229.100); **4** Thermochimica Acta 449 (2006) 67-72; **5** www.zirchrom.com/organic.htm; **6** Croat. Chem. Acta 87 (2014), 385; **7** CAS SciFinder™ (predicted property); **8** Experimentelle Bestimmung durch Halbtitration mit 1N HCl; **9** Wikipedia (deutsche und englische Websites), Beachtung des nivellierenden Effekts von Wasser.

Wie bereits oben ausgeführt zeigt EMIM-BF4 ohne Zusatz eine saure Reaktion mit Wasser und einen weiteren pH-Wert Abfall bei Kontakt mit Wasser (Nr. 40). Auch bei anfänglichen Entfernen von Protonen mittels Ionenaustauscher sinkt der pH-Wert drastisch (Nr. 37, ΔpH), wobei die Freisetzung von Protonen eine Hydrolyse des Anions anzeigt. Durch die Zugabe von Additiven mit einem pKs-Wert von zumindest 5 wird

Erfindungsgemäße Additive sind zumindest geeignet in der vorliegenden Versuchsanordnung einen pH-Wert über 4, bevorzugt über 5, zu stabilisieren, und somit sicherzustellen, dass der Anteil an HF (% HF) in Bezug auf die Gesamtmenge Fluorid unter 14,8 %, beziehungsweise unter 1,5 % liegt, berechnet aus der Henderson-Hasselbalch Gleichung und dem pKs-Wert von 3,17 für HF (% HF).

Ein pH-Grenzwert von 4 ist auch von praktischer Relevanz, da gemäß den Erfahrungen der Erfinder im Gasraum über einer ionischen Flüssigkeit mit den Anionen $BF_4^-$ oder $PF_6^-$ mittels HF-Messröhrchen (Dräger Nr. 810325, https://www.draeger.com) Fluorwasserstoff nachgewiesen werden kann, wenn der pH-Wert ≤ 4 ist.

Für 2,6-Di-tert-butylpyridin (Nr. 39) ist der $pK_S$-Wert mit 3,58 zu niedrig, um eine dauerhafte Erhöhung des pH-Werts über pH = 4 zu erreichen.

Zusätzlich stellt sich heraus, dass nicht alle Basen mit $pK_S$-Werten von zumindest 5 gleich gut geeignet sind. So zeigt LiOH trotz des hohen pKs-Wert keine dauerhafte Erhöhung des pH-Werts der Zusammensetzung mit Wasser (Nr.38). Auch andere Additive auf Hydroxid-Basis führen trotz vergleichsweise hohen $pK_S$-Werten zu einer dauerhaften Stabilisierung des pH-Werts auf vergleichsweise niedrigen Werten, so dass vermutet wird, dass hier die Hydrolyse des Anions möglicherweise verstärkt wird (Nr. 14, Nr. 29, ΔpH).

Besonders geeignet sind hingegen die Stickstoffbasen und davon insbesondere die Imidazole (Nr. 4, 5 und 7), die Transalkylierungsprodukte des Kation EMIM sind, oder zumindest ähnlich sind.

**Beispiel 2 - Untersuchung von verschiedenen Additiven in Zusammensetzungen mit der ionischen Flüssigkeit 1-Butyl-3-methylimidazolium-hexafluorophosphat**

Die hydrophobe ionische Flüssigkeiten 1-Butyl-3-methylimidazolium-hexafluorophosphat ("BMIM-PF6", CAS 174501-64-5) wurde analog zu Beispiel 1 mit je 10mol% einer Reihe verschiedener basischer Additive und 50 m% Wasser versetzt und bis zum konstanten pH-Wert der wässrigen Phase bei 80 °C gerührt (2-Phasensystem), was nach 216 Stunden für alle Reaktionsgemische der Fall war. Der pH-Wert wurde dabei immer in der oberen, leicht absetzbaren,

wässrigen Phase bestimmt.

Tabelle 3 und 4 fassen die Ergebnisse von BMIM-PF6 unter Zusatz von 10 mol% verschiedener Basen und 50 m% Wasser bei 80 °C, sowie Löslichkeit der Additive in BMIM-PF6 ohne Wasserzusatz nach 24h bei 80 °C (Sol.), zusammen.

Tabelle 3:

| Nr. | Substanz | CAS | pKs in H2O | Quelle pKs | pH Start (0h) | pH Ende (216h) | ΔpH | %HF |
|---|---|---|---|---|---|---|---|---|
| 1 | KOH | 1310-58-3 | 14,00 | 9 | 11,78 | 13,03 | -1,25 | 0,000 |
| 2 | LiOH | 1310-65-2 | 14,00 | 9 | 11,93 | 12,18 | -0,25 | 0,000 |
| 3 | N,N,N',N'-Tetramethylguanidin | 80-70-6 | 13,60 | 6 | 12,95 | 11,34 | 1,61 | 0,000 |
| 4 | n-Butylamin | 109-73-9 | 10,67 | 4 | 11,92 | 11,24 | 0,68 | 0,000 |
| 5 | Dibutylamin | 111-92-2 | 11,07 | 4 | 11,62 | 11,16 | 0,46 | 0,000 |
| 6 | 1-Methylpyrrolidin | 120-94-5 | 10,32 | 5 | 11,84 | 11,09 | 0,74 | 0,000 |
| 7 | K2CO3 | 584-08-7 | 10,40 | 2 | 11,15 | 11,08 | 0,07 | 0,000 |
| 8 | Na2CO3 | 497-19-8 | 10,40 | 2 | 10,92 | 10,88 | 0,04 | 0,000 |
| 9 | TMEDA | 110-18-9 | 8,97 | 2 | 11,30 | 10,56 | 0,74 | 0,000 |
| 10 | L-Arginin | 74-79-3 | 12,10 | 2 | 11,03 | 10,06 | 0,97 | 0,000 |
| 11 | Tributylamin | 102-82-9 | 9,05 | 4 | 9,98 | 9,99 | 0,00 | 0,000 |
| 12 | Tris-Base | 77-86-1 | 8,07 | 2 | 10,58 | 9,44 | 1,14 | 0,000 |
| 13 | Triethanolamin | 102-71-6 | 7,74 | 2 | 10,48 | 9,21 | 1,27 | 0,000 |
| 14 | Tributylmethylammonium methylcarbonat | 120256-45-3 | 6,50 | 8 | 8,49 | 8,75 | -0,26 | 0,000 |
| 15 | 1-Methylimidazol | 616-47-7 | 7,21 | 1 | 10,20 | 8,49 | 1,72 | 0,000 |
| 16 | 1-Buty-3-methylimidazolium methylcarbonat | 916850-37-8 | 6,50 | 8 | 8,55 | 8,46 | 0,08 | 0,001 |
| 17 | Imidazol | 288-32-4 | 6,96 | 1 | 9,85 | 8,44 | 1,42 | 0,001 |
| 18 | 1-Ethylimidazol | 7098-07-9 | 7,26 | 1 | 10,15 | 8,36 | 1,79 | 0,001 |
| 19 | 2,6-Lutidine | 108-48-5 | 6,60 | 2 | 9,66 | 7,72 | 1,94 | 0,003 |
| 20 | Trioctylamin | 1116-76-3 | 7,18 | 3 | 9,90 | 7,34 | 2,56 | 0,007 |
| 21 | Pyridin | 110-86-1 | 5,23 | 2 | 8,80 | 6,58 | 2,23 | 0,039 |
| 22 | kein Additiv | - | | | 6,61 | 1,04 | 5,57 | 99,27 |

Tabelle 4:

| Nr. | Substanz | Sol. | $\Delta n_D^{20}$ | Änderung Aspekt & Farbe | Änderung HPLC, IC |
|---|---|---|---|---|---|
| 1 | KOH | (±) | -0,0014 | klar, leicht gelb, unverändert | unverändert |
| 2 | LiOH | (±) | -0,0010 | trüb, gelb, unverändert | unverändert |
| 3 | N,N,N',N'-Tetramethylguanidin | + | -0,0009 | klar, leicht gelb, unverändert | unverändert |
| 4 | n-Butylamin | + | -0,0019 | klar, leicht gelb nach trübe, leicht gelb | unverändert |

(fortgesetzt)

| Nr. | Substanz | Sol. | $\Delta n_D^{20}$ | Änderung Aspekt & Farbe | Änderung HPLC, IC |
|---|---|---|---|---|---|
| 5 | Dibutylamin | + | -0,0014 | klar, leicht gelb nach trübe, leicht gelb | unverändert |
| 6 | 1-Methylpyrrolidin | + | 0,0000 | klar, leicht gelb nach trübe, leicht gelb | unverändert |
| 7 | K2CO3 | ± | -0,0011 | trüb, gelb, unverändert | unverändert |
| 8 | Na2CO3 | ± | -0,0014 | trüb, gelb, unverändert | unverändert |
| 9 | TMEDA | + | -0,0005 | klar, gelb nach trübe, gelb | unverändert |
| 10 | L-Arginin | ± | -0,0011 | trüb, gelb, unverändert | unverändert |
| 11 | Tributylamin | - | -0,0025 | klar, leicht gelb nach trübe, leicht gelb | unverändert |
| 12 | Tris-Base | ± | -0,0016 | klar, leicht gelb nach trübe, leicht gelb | unverändert |
| 13 | Triethanolamin | + | -0,0023 | klar, gelb nach trübe, gelb | unverändert |
| 14 | Tributylmethylammonium methylcarbonat | + | -0,0037 | klar, leicht gelb nach trübe, leicht gelb | unverändert |
| 15 | 1-Methylimidazol | + | 0,0000 | klar, leicht gelb, unverändert | unverändert |
| 16 | 1-Butyl-3-methylimidazolium methylcarbonat | + | -0,0065 | klar, leicht gelb nach trübe, leicht gelb | unverändert |
| 17 | Imidazol | + | -0,0005 | klar, leicht gelb, unverändert | unverändert |
| 18 | 1-Ethylimidazol | + | -0,0007 | klar, leicht gelb, unverändert | unverändert |
| 19 | 2,6-Lutidine | + | -0,0003 | klar, leicht gelb, unverändert | unverändert |
| 20 | Trioctylamin | - | -0,0008 | klar, gelb nach trübe, gelb | unverändert |
| 21 | Pyridin | + | -0,0003 | klar, leicht gelb, unverändert | unverändert |
| 22 | kein Additiv | | -0,0243 | klar, gelb nach trübe, gelb (Niederschlag) | unverändert |

Abkürzungen und Quellenangaben siehe **Fehler! Verweisquelle konnte nicht gefunden werden.** und 2.

BMIM-PF6 reagiert zunächst weniger sauer als EMIM-BF4. Dieser Unterschied zwischen Hexalfuorophosphaten und Tetrafluoroboraten wurde bereits beschrieben ("Hydrolysis of Tetrafluoroborate and Hexafluorophosphate Counter Ions in Imidazolium-Based Ionic Liquids"; Freire, Mara G.; Coutinho, Joao A. P. et al., J. Phys. Chem. A (2010), 114, 3744-3749).

Dennoch zeigt auch BMIM-PF6 in einer Zusammensetzung mit Wasser einen starken Abfall des pH-Werts bevor sich dieser stabilisiert (Nr. 22). Bei diesem pH-Wert liegt freigesetztes Fluorid zu 99,27 % als HF vor. Der pH-Wert kann hier durch die Zugabe von verschiedenen Basen erfolgreich auf einen Wertebereich stabilisiert werden, bei dem der % HF-Wert unter 5 % liegt.

**Beispiel 3 - Transalkylierungsreaktionen zwischen ionischen Flüssigkeiten und Stickstoffbasen**

**Beispiel 3.1:** 1-Ethyl-3-methylimidazolium-tetrafluoroborat mit 1-Ethylimidazol

5,20 g 1-Ethyl-3-methylimidazolium-tetrafluoroborat (EMIM-$BF_4$, CAS 143314-16-3) wurde mit 5 m% (0,27g) 1-Ethylimidazol (CAS 7098-07-9) versetzt und in einem druckfesten Reaktionsgefäß ($p_{max}$ 20 bar) mit Schraubverschluss, Teflondichtung und Teflon-Magnetrührer in einem thermostatisierten Aluminiumblock bei 200 ± 0,2 °C für 7 Tage gerührt. Danach wurde die Probe mittels HPLC analysiert.

Tabelle 5 zeigt die Transalkylierungsprodukte von EMIM-$BF_4$ mit 5 m% 1-Ethylimidazol nach 7d bei 200 °C,

wie sie mittels HPLC analysiert wurden.

Tabelle 5:

| Substanz | Retentionszeit [min] | Relative Fläche [%] |
|---|---|---|
| 1-Methylimidazol (MI) | 7,67 | 0,92 |
| 1-Ethylimidazol (EI) | 8,43 | 7,15 |
| 1,3-Dimethylimidazolium ($DMIM^+$) | 9,22 | 1,71 |
| 1-Ethyl-3-methylimidazolium ($EMIM^+$) | 10,15 | 87,25 |
| 1,3-Diethylimidazolium ($DEIM^+$) | 10,97 | 2,97 |

Das Analysenergebnis zeigte deutlich die stattgefundene Transalkylierung, welche durch die zugegeben Base 1-Ethylimidazol ausgelöst worden ist (siehe Fehler! Verweisquelle konnte **nicht gefunden werden.).** Das nun thermisch equilibrierte Gemisch aus drei ionischen Flüssigkeiten und zwei Basen verhält sich aber gemäß den Erfahrungen der Erfinder hinsichtlich wesentlicher physikochemischer Daten nahezu unverändert, weil die grundsätzliche Klasse der ionischen Flüssigkeiten (1,3-Dialkylimidazolium tetrafluoroborate) und der Basen (Alkylimidazole) unverändert blieb, lediglich die Alkylgruppen neu verteilt worden sind und die Alkylgruppen sich durch nur eine $CH_2$-Gruppe voneinander unterscheiden. So haben die drei entstehenden 1,3-Dialkylimidazolium tetrafluoroborate alle eine Schmelzpunkt im Bereich von 10 bis ungefähr 100 °C, nämlich EMIM-BF4: 13 °C; DEIM-BF4: 38-40 °C (alle Werte verfügbar über CAS SciFinder™ Datenbank); DMIM ungefähr 100 °C gemäß ("The phase behaviour of 1-alkyl-3-methylimidazolium tetrafluoroborates; ionic liquids and ionic liquid crystals" Holbrey, J.D.; Seddon, K.R.; J. Chem. Soc., Dalton Trans., 1999, 2133-2139).

Die verwendete Base 1-Ethylimidazol und die entstehende Base 1-Ethylimidazol zeichnen sich insbesondere dadurch aus, dass sie beide formal die Ausgangsstoffe der Synthese des 1,3-Dialkylimidazolium-Kations sind (Alkylierung in einer *Menschutkin*-Reaktion). Es handelt sich also bei 1-Ethylimidazol bzw. 1-Ethylimidazol um Dealkylierungsprodukte des Kations die formal aus dem Kation durch Dealkylierung abgeleitet werden können (reverse *Menschutkin*-Reaktion).

**Beispiel 3.2:** 1-Ethyl-3-methylimidazolium-tetrafluoroborat mit 1-Butylimidazol

4,07 g 1-Ethyl-3-methylimidazolium-tetrafluoroborat (EMIM-BF4, CAS 143314-16-3) wurde mit 5 m% (0,21g) 1-Butylimidazol (CAS 4316-42-1) versetzt und analog wie in Experiment 3.1 behandelt (thermostatisiert bei 200 $\pm$ 0,2 °C für 7 Tage).

Danach wurde die Probe mittels HPLC analysiert und die in Tabelle 6 gelisteten Substanzen als Transalkylierungsprodukte:

Tabelle 6:

| Substanz | Retentionszeit [min] | Relative Fläche [%] |
|---|---|---|
| 1-Ethylimidazol (EI) | 8,34 | 2,68 |
| 1-Ethyl-3-methylimidazolium ($EMIM^+$) + 1-Butylimidazol (BI); Signale überlagert | 10,18 | 92,55 |
| 1-Butyl-3-methylimidazolium ($BMIM^+$) | 12,78 | 4,77 |

Die Reaktionen sind in Fig. 3A dargestellt, das Chromatogramm ist in Fig. 3B wiedergegeben. Das Analysenergebnis zeigte deutlich die stattgefundene Transalkylierung, welche durch die zugegebene Base 1-Butylimidazol ausgelöst worden. Da 1-Butylimidazol aber noch immer zur gleichen Klasse der 1-Alkylimidazole gehört, sind die entstandenen ionischen Flüssigkeiten noch immer in derselben Klasse der 1,3-Dialkylimidazolium tetrafluoroborate und das neu entstandene, thermisch equilibrierte Gemisch zeigt wie das aus Beispiel 3.1. wenig veränderte physikochemische Eigenschaften. Auch BMIM-BF4 hat einen sehr niedrigen Schmelzpunkt von -17 °C (Wert verfügbar über CAS SciFinder™ Datenbank).

**Beispiel 3.2:** 1-Ethyl-3-methylimidazolium-tetrafluoroborat mit 1-Butylpyrrolidin

5,15 g 1-Ethyl-3-methylimidazolium-tetrafluoroborat (EMIM-$BF_4$, CAS 143314-16-3) wurde mit 5 m% (0,27 g) 1-Butylpyrrolidin (CAS 767-10-2) versetzt und analog wie in Experiment 3.1 behandelt. Danach wurde die Probe mittels HPLC analysiert und zeigte die in Tabelle 7 gezeigte Zusammensetzung:

Tabelle 7:

| Substanz | Retentionszeit [min] | Relative Fläche [%] |
|---|---|---|
| 1-Ethylimidazol (EI) | 8,41 | 2,06 |
| 1 -Ethyl-3-methylimidazolium ($EMIM^+$) | 10,12 | 70,23 |
| 1-Butyl-3-methylimidazolium ($BMIM^+$) | 12,54 | 27,71 |

Das Analysenergebnis zeigte deutlich die stattgefundene Transalkylierung. Das Produkt BMIM+ kann formal nicht aus dem 1-Ethyl-3-methylimidazolium Kation durch Dealkylierung abgeleitet werden (reverse *Menschutkin*-Reaktion). Die Transalkylierung wurde also durch die zugegebene Base 1-Butylpyrrolidin ausgelöst (siehe Fig. 4). Mögliche weitere Produkte 1-Butylpyrrolidin und 1,1-Dialkylpyrrolidinium sind nicht UV-aktiv und wurden daher nicht detektiert.

Das neu entstandene, thermisch equilibrierte Gemisch besitzt potentiell eine nahezu unkontrollierbare Vielzahl verschiedener 1,3-Dialkylimidazolium- und 1,1-Dialkylpyrrolidinium-Kationen, sowie 1-Alkylimidazole und 1-Alkylpyrrolidine, von denen in der Praxis mittels HPLC-UV insbesondere eine große Konzentration an 1-Butyl-3-methylimidazolium nachgewiesen werden konnte (Fig. 4). Die Alkylpyrrolidiniumkationen führen in Tetrafluoroborat-Verbindungen, im Gegensatz zu den 1,3-Dialkylimidazoliumverbindungen, zu wesentlich höheren Schmelzpunkten. So hat bereits 1-Butyl-1-methylpyrrolidium tetrafluoroborat einen Schmelzpunkt von 150 °C; für 1-Ethyl-1-methylpyrrolidium tetrafluoroborat wird ein Schmelzpunkt von 280 oder 294 °C angegeben und 1,1 -Dimethylpyrrolidium tetrafluoroborat zersetzt sich bei 340 °C ohne zu schmelzen (alle Angaben verfügbar über CAS SciFinder™ Datenbank). Entsprechend muss für die entstehende Mischung eine Veränderung des Verhaltens gegenüber der ursprünglichen Zusammensetzung erwartet werden.

**Beispiel 4** - **Untersuchung von EMIM-BF mit Zusatz von Ethylimidazol**

20g 1-Ethyl-3-methylimidazolium-tetrafluoroborat (EMIM-BF4, CAS 143314-16-3) wurden mit Reinstwasser auf 10 m% verdünnt (Wassergehalt 90 m%, entspricht rund 99 mol% Wasser). Durch Behandlung mit ca. 5g Ionenaustauscher DOWEX® MWA-1 (freie $NH_2$-Base) wurde die wässrige Lösung entsäuert (Entfernung von HF) und der Ionenaustauscher durch dekantieren abgetrennt. Die so behandelte Probe wurde mit 10 mol% (bezogen auf EMIM-BF4) 1-Ethylimidazol (EI, CAS 7098-07-9) versetzt. Eine Referenzprobe wurde gleich behandelt, allerdings ohne Zusatz von 1-Ethylimidazol. Die beiden Proben wurden analog zu Beispiel 1 in verschlossenen Teflongefäßen bei 80 $\pm$ 0,1 °C thermostatisiert und der pH Wert der Zusammensetzung in regelmäßigen Abständen gemessen (s. Tabelle 8).

Tabelle 8:

| time [h] | pH EMIM-BF4 ohne EI | pH EMIM-BF4 10 mol % EI |
|---|---|---|
| 0,00 | 6,9 | 8,5 |
| 0,25 | 6,8 | 8,3 |
| 0,50 | 6,1 | 8,1 |
| 0,75 | 5,2 | 8,0 |
| 1,00 | 4,8 | 7,8 |
| 2,00 | 4,2 | 7,3 |
| 3,00 | 3,18 | 6,6 |
| 4,00 | 2,9 | 6,3 |
| 7,00 | 2,8 | 5,1 |
| 24,00 | 2,8 | 3,8 |

(fortgesetzt)

| time [h] | pH<br>EMIM-BF4 ohne EI | pH<br>EMIM-BF4 10 mol % EI |
|---|---|---|
| 48,00 | 2,8 | 3,7 |

Bereits nach 2 Stunden konnten im Gasraum des Teflongefäßes der nicht addivierten Probe mittels HF-Messröhrchen (Dräger Nr. 810325, https://www.draeger.com) ansteigende Konzentrationen an Fluorwasserstoff nachgewiesen werden, während in der additiveren Probe kein HF nachgewiesen werden konnte.

**Beispiel 5 - Untersuchung von EMIM-BF mit Zusatz von Ethylimidazol bei Kontakt mit Alkohol**

20g 1-Ethyl-3-methylimidazolium-tetrafluoroborat (EMIM-BF4, CAS 143314-16-3) wurde mit Methanol puriss. p.a. auf 50 m% verdünnt (Methanolgehalt 50 m%, ca. 85 mol%). Durch Behandlung mit ca. 5g Ionenaustauscher DOWEX® MWA-1 (freie $NH_2$-Base) wurde die methanolische Lösung entsäuert (Entfernung von HF) und der Ionenaustauscher durch dekantieren abgetrennt. Die so behandelte Probe wurde mit 10 mol% (bezogen auf EMIM-BF4) 1-Ethylimidazol (EI, CAS 7098-07-9) versetzt. Eine Referenzprobe wurde gleich behandelt, allerdings ohne Zusatz von 1-Ethylimidazol. Die beiden Proben wurden analog zu Beispiel 1 in verschlossenen Teflongefäßen bei 80 ± 0,1 °C thermostatisiert und der pH Wert in regelmäßigen Abständen mittels einer Metrohm Solvotrode (Nr. 6.0229.100) gemessen. Die Ergebnisse der Solvolyse von 50 m% EMIM-BF4 in Methanol bei 80 °C, ohne und mit Zusatz von 10 mol% 1-Ethylimidazol (EI) sind in Tabelle 9 gezeigt.

Tabelle 9:

| time [h] | pH<br>EMIM-$BF_4$ ohne EI | pH<br>EMIM-$BF_4$ 10 mol % EI |
|---|---|---|
| 0 | 5,4 | 8,0 |
| 1 | 3,9 | 7,2 |
| 2 | 3,7 | 7,2 |
| 3 | 3,7 | 7,1 |
| 5 | 3,6 | 7,1 |

Die unbehandelte Probe änderte zu Beginn den pH-Wert so rasch, dass wenige Minuten nach Abtrennung des Ionenaustauschers bei Raumtemperatur der pH Wert von 6,3 auf 5,4 gesunken war. Nach 1 Stunde konnten im Gasraum des Teflongefäßes der nicht addivierten Probe mittels HF-Messröhrchen (Dräger Nr. 810325, https://www.draeger.com) erhebliche Konzentrationen an Fluorwasserstoff nachgewiesen werden, während in der additiveren Probe kein HF nachgewiesen werden konnte.

**Beispiel 6** - **Untersuchung von EMIM-BF mit Zusatz von Ethylimidazol**

20g 1-Ethyl-3-methylimidazolium-tetrafluoroborat (EMIM-BF4, CAS 143314-16-3) wurden mit Reinstwasser auf 90 m% verdünnt (Wassergehalt 10 m%, entspricht rund 55 mol% Wasser). Durch Behandlung mit ca. 5g Ionenaustauscher DOWEX® MWA-1 (freie $NH_2$-Base) wurde die wässrige Lösung entsäuert (Entfernung von HF) und der Ionenaustauscher durch dekantieren abgetrennt. Die so behandelte Probe wurde mit 10 mol% (bezogen auf EMIM-BF4) 1-Ethylimidazol (EI, CAS 7098-07-9) versetzt. Eine Referenzprobe wurde gleich behandelt, allerdings ohne Zusatz von 1-Ethylimidazol. Die beiden Proben wurden analog zu Beispiel 1 in verschlossenen Teflongefäßen bei 80 ± 0,1 °C thermostatisiert. In regelmäßigen Abständen wurden Teilproben gezogen und der pH50 durch Verdünnen mit Wasser auf 50 %m gemessen. Tabelle 10 zeigt die Ergebnisse für die pH50 Werte der Teilproben aus den beiden Ansätzen.

Tabelle 10:

| time [h] | pH50<br>EMIM-BF4 ohne EI | pH50<br>EMIM-BF4 10 mol% EI |
|---|---|---|
| 0 | 7,1 | 9,4 |

(fortgesetzt)

| time [h] | pH50 EMIM-BF4 ohne EI | pH50 EMIM-BF4 10 mol% EI |
|---|---|---|
| 1 | 6,8 | 9,1 |
| 2 | 6,8 | 9,2 |
| 3 | 6,4 | 8,9 |
| 24 | 3,1 | 8,6 |
| 47 | 3,2 | 8,0 |
| 70 | 3,3 | 7,1 |
| 166 | 3,3 | 7,2 |

Auch hier ist der Effekt des Additives EI auf die pH-Wert Entwicklung klar erkennbar.

**Beispiel 7** - **Untersuchung von DEME-BF4 mit 1-Ethylimidazol**

20g N,N-Diethyl-N-methyl-N-(2-methoxyethyl)ammonium tetrafluoroborate (DEME-BF4, CAS 464927-72-8) wurden mit Reinstwasser auf 50 m% verdünnt (Wassergehalt 50 m%, entspricht rund 93 mol% Wasser). Durch Behandlung mit ca. 1g Ionenaustauscher DOWEX® MWA-1 (freie $NH_2$-Base) wurde die wässrige Lösung entsäuert (Entfernung von HF) und der Ionenaustauscher durch dekantieren abgetrennt. Zwei so behandelte Proben wurde mit je 10 mol% (bezogen auf DEME-BF4) 1-Ethylimidazol (EI, CAS 7098-07-9) bzw. Triethylamin (TEA, CAS 121-44-8) versetzt. Eine weitere Referenzprobe wurde gleich behandelt, allerdings ohne Zusatz einer Base. Die drei Proben wurden analog zu Beispiel 1 in verschlossenen Teflongefäßen bei 80 ± 0,1 °C thermostatisiert und in regelmäßigen Abständen der pH-wert gemessen (s. Tabelle 11).

Tabelle 11:

| time [h] | pH DEME-BF4 ohne EI | pH DEME-BF4 10 mol% EI | pH DEME-BF4 10 mol% TEA |
|---|---|---|---|
| 0 | 7,5 | 9,4 | 12,1 |
| 1 | 2,4 | 8,4 | 11,9 |
| 4 | 2,6 | 8,0 | 11,8 |
| 7 | 2,5 | 7,9 | 11,6 |
| 24 | 2,4 | 6,2 | 8,5 |
| 48 | 2,4 | 5,7 | 6,0 |
| 72 | 2,4 | 5,7 | 5,3 |
| 144 | 2,3 | 5,5 | 5,3 |
| 192 | 2,4 | 5,6 | 5,2 |

**Beispiel 8** - **Untersuchung von TBMP-BF4 mit 1-Ethylimidazol**

Tributylmethylphosphonium tetrafluoroborat (TBMP-BF4, CAS 86042-82-2) ist eine hydrophobe ionische Flüssigkeit mit einem Schmelzpunkt von 96 - 100 °C. 10g TBMP-BF4 wurden mit 0,35g (10 mol%) 1-Ethylimidazol (EI, CAS 7098-07-9) versetzt; zu dieser Einwaage wurden nun 10,35g Reinstwasser (Massenverhälnis 1:1, entspricht rund 94 mol%) zugesetzt. Eine Referenzprobe wurde in gleicher Weise aber ohne Zusatz von EI hergestellt. Die beiden Proben wurden analog zu Beispiel 2 in verschlossenen, druckfesten Teflongefäßen bei 120 ± 0,1 °C thermostatisiert und in regelmäßigen Abständen der pH-Wert der überstehenden wässrigen Phase gemessen (s. Tabelle 12).

Tabelle 12:

| time [h] | pH TBMP-BF4 ohne EI | pH TBMP-BF4 10 mol% EI |
|---|---|---|
| 0 | 6,0 | 8,6 |
| 1 | 5,0 | 7,5 |
| 5 | 4,2 | 7,1 |
| 9 | 3,8 | 6,6 |
| 24 | 3,6 | 5,8 |
| 48 | 3,5 | 5,5 |
| 72 | 3,6 | 5,7 |

**Beispiel 9** - **Untersuchung von Chol-$PF_6$ mit 1-Ethylimidazol**

Cholinium-hexafluorophosphat (Chol-$PF_6$, CAS 1040887-91-9) ist ein hydrophobes organisches Salz mit einem Schmelzpunkt von 270 °C. 15g Chol-$PF_6$ wurden mit 0,59 g (10 mol%) 1-Ethylimidazol (EI, CAS 7098-07-9) versetzt; zu dieser Einwaage wurden nun 15,59 g Reinstwasser (Massenverhälnis 1:1, entspricht rund 93 mol%) zugesetzt. Eine Referenzprobe wurde in gleicher Weise aber ohne Zusatz von EI hergestellt. Die beiden Proben wurden analog zu Beispiel 2 in verschlossenen, druckfesten Teflongefäßen bei 120 $\pm$ 0,1 °C thermostatisiert und in regelmäßigen Abständen der pH-Wert der überstehenden wässrigen Phase gemessen (s. Tabelle 13).

Tabelle 13:

| time [h] | pH Chol-$PF_6$ ohne Additiv | pH Chol-$PF_6$ 10 mol% EI |
|---|---|---|
| 0 | 6,0 | 7,3 |
| 4 | 5,9 | 7,1 |
| 6 | 5,9 | 7,0 |
| 28 | 5,1 | 6,7 |
| 96 | 4,9 | 6,7 |
| 120 | 4,9 | 6,6 |
| 144 | 5,0 | 6,6 |

**Beispiel 10 - Korrosionsbestimmung**

3.500g 1-Ethyl-3-methylimidazolium-tetrafluoroborat (EMIM-BF4, CAS 143314-16-3) wurden mit 4 m% 1-Ethylimidazol (EI, CAS 7098-07-9) versetzt und zusammen mit Materialproben zur Korrosionsbestimmung in einem 20 Liter Rührautoklaven aus Edelstahl 1.4571 für insgesamt 60 Tage unter Argon-Atmosphäre auf 200 °C erhitzt. In regelmäßigen Abständen wurden Proben gezogen und analysiert (s. Tabelle 14).

Tabelle 14:

| time [d] | F- [ppm] | BF4- als EMIM- BF4 [wt%] | pH50 | $n_D^{20}$ | $H_2O$ [wt%] | HPLC |
|---|---|---|---|---|---|---|
| | (IC) | (IC) | | | (KFT) | |
| 0 | 7381 | 94,1 | 7,2 | 1,4185 | 0,512 | |
| 1 | 7406 | 95,1 | 7,0 | 1,4185 | 0,617 | unverändert |
| 5 | 6471 | 95,0 | 7,1 | 1,4180 | 0,614 | unverändert |
| 15 | 6772 | 94,9 | 7,1 | 1,4180 | 0,593 | unverändert |
| 32 | 5826 | 94,8 | 7,1 | 1,4185 | 0,583 | unverändert |

(fortgesetzt)

| time [d] | F- [ppm] | BF4- als EMIM- BF4 [wt%] | pH50 | $n_D^{20}$ | $H_2O$ [wt%] | HPLC |
|---|---|---|---|---|---|---|
| | (IC) | (IC) | | | (KFT) | |
| 60 | 4803 | 95,8 | 7,3 | 1,4175 | 0,588 | unverändert |

Tabelle 14 zeigt keinerlei auffällige Veränderungen an den wesentlichen Parametern, obwohl die Farbe der additivierten ionischen Flüssigkeit im Laufe der Zeit von gelb nach dunkelbraun wechselte. Die in der HPLC beobachtbaren Veränderungen entsprachen lediglich den zu erwartenden Transalkylierungsreaktionen (siehe Beispiel 3). Die beobachtete Abnahme des Fluoridwertes wurde auf Bildung von Eisenfluoriden der Materialproben zurückgeführt.

Wie weiter oben bereits erwähnt, wurden zur Messung von Korrosion zusätzlich über die gesamte Zeitdauer Materialproben in Form von Testcoupons 25 mm x 10 mm x 1 mm eingelagert (Dreifachbestimmung). Die Korrosionsraten wurden durch exaktes Auswiegen der Proben vor und nach der Einlagerung (Probenkörper jeweils entfettet und gereinigt mit Isopropanol und Reinstwasser im Ultraschallbad, getrocknet bei 150 °C im Trockenschrank), Umrechnung des Massenverlusts über die Dichte des Materials und die Zeitdauer des Tests auf mm/a bestimmt.

Tabelle 15:

| Materialnummer | 1.0570 | 1.4301 | 1.4571 | Reinkupfer | 2.4360 |
|---|---|---|---|---|---|
| Korrosion Δ d/t [mm/Jahr] | 0.08 | 0.03 | < 0.01 | 0.02 | 0.01 |

**Fehler! Verweisquelle konnte nicht gefunden werden.** zeigt, dass selbst für einen niederlegierten Werkzeugstahl 1.0570 (St52) eine noch tolerierbare Korrosion zu erkennen ist, welche durch Zusatz von Korrosionsschutzadditiven weiter verbessert werden kann. Höher legierte Stähle und die Buntmetalle Kupfer und 2.4360 (eine Ni-Cu Legierung) zeigten sehr gute Werte. Sämtliche Testcoupons zeigten visuell keine oder nur geringe Verfärbung und unter dem Mikroskop keinerlei Lochfraß- oder Spaltkorrosion, d.h. die beobachtete Korrosion ist lediglich eine gut beherrschbare Flächenkorrosion. Dies ist insbesondere bemerkenswert, da für die ionische Flüssigkeit alleine, die einen pH 50 von ca. 2,5 zeigt, eine starke Korrosion zu erwarten ist.

**Beispiel 11 - Elektrochemisches Fenster**

Im folgenden Beispiel wurden elektrochemische Fenster an 1-Ethyl-3-methylimidazolium-tetrafluoroborat (EMIM-BF4, CAS 143314-16-3) mittels Cyclovoltammetrie gemessen. Das verwendete EMIM-BF4 zeigte folgende, für elektrochemische Anwendungen sehr gut geeignete Qualitätssicherungsdaten (Tabelle 16).

Tabelle 16:

| Test | Resultat |
|---|---|
| Gehalt EMIM-$BF_4$ via $EMIM^+$ (HPLC) | 99.04 ± 0.24 wt% |
| Gehalt EMIM-$BF_4$ via $BF_4^-$ (Titration) | 99.34 ± 0.03 wt% |
| Gehalt $H_2O$ (Karl Fischer Coulometrie) | 49 ± 2 $ppm_w$ |
| Gehalt 1-Ethylimidazole (HPLC) | 600 ± 50 $ppm_w$ |
| Gehalt Halogenide ohne Fluorid (IC) | nicht nachweisbar (< 1 ppm) |
| Gehalt Fluorid (IC) | 0.48 ± 0.008 wt% |
| Aspekt gemäß Ph. Eur. 5.0 | klare Flüssigkeit |
| $pH_{50}$ | 2.17 |

Das EMIM-BF4 zeigte einen produktionsbedingt sauren pH50 Wert von 2,2. Dieser pH50 Wert liegt im typischen Bereich von ca. 2-3, wie man ihn auch in anderen, kommerziell erhältlichen Produkten typisch vorfindet.

Das elektrochemische Fenster wurde mit einem Autolab PGSTAT204 Potentiostat/Galvanostat bei Raumtemperatur unter ständiger Inertgas-Spülung mit trockenem Argon gemessen. Als Arbeits- und Gegenelektrode wurde Glaskohlenstoff (Glassy Carbon) verwendet. Als Quasi-Referenzelektrode (QRE) diente ein Platindraht. Der Abstand zwischen Arbeitselektrode und QRE betrug ca. 0,7 cm. Die Arbeitselektrode hatte einen Radius von 2 mm. Der Potentialvorschub wurde mit 0,01 V/s gewählt, die automatische Strombegrenzung auf 100 µA - 100 nA festgelegt. Die an der QRE anliegenden Umkehrpunkte wurden mit $\pm 1.25 \cdot 10^{-4}$ A definiert. Alle Elektroden wurden vor der Messung poliert, mit Reinstwasser und Isopropanol gespült und anschließend sorgfältig getrocknet. Proben wurden mit sterilen Spritzen über ein Septum aus inertisierten Glasvials transferiert. Referenzwert für das elektrochemische Fenster von EMIM-BF4: $\Delta E$ = 4.4 V (M. Lipsztajn und R. A. Osteryoung, J. Electrochem. Soc. 1983 130(9), 1968-1969).

In einer ersten Messung wurde nun von dem weiter oben beschriebenen EMIM-BF4 ohne Zugabe einer Base ein Cyclovoltammogramm (CV) aufgenommen (Fig. 5).

Das gemessene CV (siehe Fig. 5) zeigt ein typisches elektrochemisches Fenster von 4,58 V. Nahe der anodischen und kathodische Zersetzungspotentiale des EMIM-BF4 (2,03 V bzw. - 2,55 V) zeigen sich elektrochemische Signale der Zersetzung von Verunreinigungen in Form von kleinen Peaks bei ca. -2,3 V und 1,9 V, welche Wasser zugeordnet werden können. Der Peak bei ca. -1,0 V zeigt die Oxidation der bei -2,55 V aus der ionischen Flüssigkeit durch Reduktion entstandenen Zersetzungsprodukte.

Das so verwendete EMIM-BF4 zeigt mit 2,2 einen deutlich sauren, typischen pH50 Wert. pHaktive und somit "volatile" Protonen sind aber in elektrochemischen Anwendungen problematisch, da sie elektrochemisch sehr aktiv sind und außerdem zu Problemen mit Materialverträglichkeiten (Korrosion) führen können. Es wäre also von Vorteil, wenn solche Protonen gebunden werden könnten, sodass sie elektrochemisch zumindest teilweise oder gar nicht mehr zur Verfügung stehen.

Es wurde nun dem EMIM-BF4 1,0 m% der Superbase 1,8-Diazabicyclo[5.4.0]undec-7-en (DBU, CAS 6674-22-2) zugesetzt, worauf der nun gemessene pH50 = 9,7 betrug. Aufgrund der sehr hohen Bebenstärke wurde erwartet, dass die an DBU gebundenen Protonen nicht mehr elektrochemisch aktiv sind. Es wurde nun in einer zweiten Messung ein CV unter identen Bedingungen aufgenommen (s. Fig. 6).

Das CV (siehe Fig. 6) zeigt ein um immerhin 0,18 V erweitertes elektrochemisches Fenster von 4,76 V sowie nahezu verschwundenen Peaks am kathodischen und anodischen Ende bei -2,3 V und 1,9 V. Der Peak bei -1,0 V ist ebenfalls verschwunden, was darauf schließen lässt, das die nun bei -2,3 V entstehenden Zersetzungsprodukte nicht mehr oxidiert werden können bzw. gar nicht mehr entstehen. Weiters fällt auf, dass weder die zugesetzte Base DBU noch die daraus entstandene protonierte Base $DBUH^+$ innerhalb des gemessenen, großen Potentialbereichs in EMIM-BF4 elektrochemisch aktiv sind, sodass die ursprünglich aktiven Protonen nunmehr nicht mehr stören und DBI ideal geeignet ist. Weiters ist aus Beispiel 1 ersichtlich, dass DBU auch als Additiv zur Stabilisierung des pH-Werts gut geeignet ist.

**Patentansprüche**

1. Zusammensetzung umfassend eine ionische Flüssigkeit der allgemeinen Formel $[A^+]$ $[B^-]$, wobei $[A^+]$ ein organisches Kation ist und $[B^-]$ ein Anion ist ausgewählt aus $BF_4^-$ und $PF_6^-$,
   **dadurch gekennzeichnet, dass** die Zusammensetzung ein Additiv umfasst, wobei das Additiv einen $pK_S$-Wert von zumindest 5 aufweist.

2. Zusammensetzung nach Anspruch 1, wobei die Zusammensetzung Wasser, zumindest einen Alkohol oder ein Gemisch davon enthält oder in Kontakt mit Wasser, zumindest einem Alkohol oder einem Gemisch davon kommen kann.

3. Zusammensetzung nach einem der Ansprüche 1 oder 2, wobei das Additiv eine Stickstoffbase ist.

4. Zusammensetzung nach einem der Ansprüche 1 bis 3, wobei das Additiv einen $pK_S$-Wert von höchstens 11,5 aufweist.

5. Zusammensetzung nach einem der Ansprüche 1 bis 4, wobei das Additiv derart ausgewählt ist, dass das Additiv ein Dealkylierungsprodukt des Kations $[A^+]$ darstellt.

6. Zusammensetzung nach einem der Ansprüche 1 bis 5, wobei das Kation $[A^+]$ der ionischen Flüssigkeit ausgewählt ist aus der Gruppe bestehend aus quaternären Ammonium, Phosphonium, Guanidinium, Pyridinium, Pyrimidinium, Pyridazinium, Pyrazinium, Piperidinium, Morpholinium, Piperazinium, Pyrrolium, Pyrrolidinium, Oxazolium, Thiazolium, Triazinium, Imidazolium, Triazolium, protonierten Guanidinium.

7. Zusammensetzung nach einem der Ansprüche 3 bis 6, wobei die Stickstoffbase ausgewählt ist aus Gruppe bestehend aus Amin, primärem Amin, sekundärem Amin, tertiärem Amin, substituiertem oder unsubstituiertem Guanidin, Pyridin, Pyrimidin, Pyridazin, Pyrazin, Piperidin, Morpholin, Piperazin, Pyrrol, Pyrrolidin, Oxazol, Thiazol, Triazin,

Imidazol, oder Triazol, bevorzugt aus der Gruppe bestehend aus tertiärem Amin, substituiertem Guanidin, unsubstituiertem Pyridin, substituiertem oder unsubstituiertem Imidazol, und substituiertem oder unsubstituiertem Pyrrolidin.

**8.** Zusammensetzung nach einem der Ansprüche 1 bis 7, wobei das Additiv aprotisch ist.

**9.** Zusammensetzung nach einem der Ansprüche 1 bis 8, wobei das Additiv bezogen auf die Zusammensetzung in einen Gewichtsanteil von höchstens 20 %, bevorzugt von höchstens 10 %, besonders bevorzugt von höchstens 5 %, vorliegt.

**10.** Zusammensetzung nach einem der Ansprüche 1 bis 9, wobei das Kation [$A^+$] der ionischen Flüssigkeit ausgewählt ist aus Guanidinium (protoniertes Guanidin); 1,1,3,3-Tetramethylguanidinium, 1,1 ,2,3,3-Pentamethylguanidinium, 1,1,2,2,3,3-Hexamethylguanidinium, Diethyldimethylammonium, Dipropyldimethylammonium, Dibutyldimethylammonium, Dihexyldimethyl-ammonium, Dioctyldimethylammonium, Triethylmethylammonium, Tripropylmethylammonium, Tributylmethylammonium, Trihexylmethylammonium, Trioctylmethylammonium, Trimethylethylammonium, Trimethylpropylammonium, Trimethylbutylammonium, Trimethylhexyl-ammonium, Trimethyloctylammonium, Tetramethylammonium, Tetraethylammonium, Tetrapropylammonium, Tetrabutylammonium, Tetrahexylammonium, Tetraoctylammonium, 2-Methoxyethyl-trimethylammonium (O-Methyl-Cholinium), 2-Methoxyethyldiethylmethylammonium (DEME), Tris-(2-Methoxyethyl)-ammonium, Tris-(2-Methoxyethyl)-methylammonium, Tris-(2-Methoxyethyl)-ethylammonium, Bis-(2-Methoxyethyl)-dimethyl-ammonium, Triallylmethylammonium, Tetramethylphosphonium, Triethylmethylphosphonium, Tripropylmethylphosphonium, Tributylmethylphosphonium, Trihexylmethylphosphonium, Trioctylmethylphosphonium, Tetradecyl(trihexyl)phosphonium, Triisobutyl(methyl)phosphonium, Ethyl-(tributyl)-phosphonium, Octyl-(tributyl)-phosphonium, N-Decyl-N-methylpyrrolidinium, N-Octyl-N-methylpyrrolidinium, N-Hexyl-N-methylpyrrolidinium N-Butyl-N-methylpyrrolidinium, N-Propyl-N-methylpyrrolidinium, N-Ethyl-N-methylpyrrolidinium, N,N-dimethylpyrrolidinium, N-Allyl-N-methylpyrrolidinium, N-Decyl-N-methylmorpholinium, N-Octyl-N-methylmorpholinium, N-Hexyl-N-methylmorpholinium N-Butyl-N-methylmorpholinium, N-Propyl-N-methylmorpholinium, N-Ethyl-N-methylmorpholinium, N,N-Dimethylmorpholinium, N-Allyl-N-methylmorpholinium, N-Decyl-N-methylpiperidinium, N-Octyl-N-methylpiperidinium, N-Hexyl-N-methylpiperidinium N-Butyl-N-methylpiperidinium, N-Propyl-N-methylpiperidinium, N-Ethyl-N-methylpiperidinium, N,N-Eimethylpiperidinium, und N-Allyl-N-methylpiperidinium, 1,3-Dimethyl-imidazolium, 1,2,3-Trimethylimidazolium, 1-Ethyl-3-methyl-imidazolium, 1-Vinyl-3-methyl-imidazolium, 1-Vinyl-2,3-dimethyl-imidazolium, 1-Butyl-3-methylimidazolium, 1-Propyl-3-methylimidazolium, 1-iso-Propyl-3-methylimidazolium, 1-Allyl-3-methylimidazolium, 1-Propyl-2,3-dimethylimidazolium, 1-iso-Propyl-2,3-dimethylimidazolium, 1-Allyl-2,3-dimethylimidazolium, 1-Ethyl-2,3-dimethylimidazolium, 1-Butyl-2,3-dimethylimidazolium, 1-Hexyl-3-methylimidazolium, 1-Octyl-3-methylimidazolium, 1-Decyl-3-methylimidazolium, 1,3-Diethylimidazolium, und 1,3-Dibutylimidazolium und den protonierten Formen der starken Basen 1,5-Diazabicyclo[4.3.0]non-5-en (DBN); 1,8-Diazabicyclo[5.4.0]undec-7-en (DBU); 1,4-Diazabicyclo-[2.2.2]-octan (DABCO®); 7-Methyl-1,5,7-triazabicyclo[4.4.0]dec-5-ene (MTBD); 1,8-Bis-(dimethylamino)-naphthalin (Proton Sponge®); N,N,N',N'-Tetramethylethylendiamin (TMEDA); 4,5-Bis-(dimethyl-amino)-fluoren; 1,8-Bis-(hexamethyltriaminophosphazenyl)naphthalin.

**11.** Zusammensetzung nach einem der Ansprüche 1 bis 10, wobei das Additiv ausgewählt ist aus der Gruppe bestehend aus 1,3,3-Tetramethylguanidin, 1,1,2,3,3-Pentamethylguanidin, Diethylmethylamin, Dipropylmethylamin, Dibutylmethylamin, Dihexylmethylamin, Trimethylamin, Triethylamin, Tripropylamin, Triallylamin, Tributylamin, Trihexylamin, Dimethylethylamin, Dimethylpropylamin, Dimethylbutylamin, Dimethylhexylamin, 2-Methoxyethyl-dimethylamin, 2-Methoxyethyl-diethylamin, Bis-(2-Methoxyethyl)-methylamin, Bis-(2-Methoxyethyl)-ethylamin, Diallylmethylamin, Dimethylbenzylamin, Methyldibenzylamin, Tribenzylamin, N,N-Diisopropylethylamin, Pyrrolidin, N-Hexylpyrrolidin N-Butylpyrrolidin, N-Propylpyrrolidin, N-Ethylpyrrolidin, N-Methylpyrrolidin, N-Allylpyrrolidin, N-Benzylpyrrolidin, N-Hexylmorpholin N-Butylmorpholin, N-Propylmorpholin, N-Ethylmorpholin, N-Methylmorpholin, N-Allylmorpholin, N-Benzylmorpholin, Morpholin, N-Hexylpiperidin, N-Butyl-piperidin, N-Propylpiperidin, N-Ethylpiperidin, N-Methylpiperidin, N-Allylpiperidin, Piperazin, $C_1$-$C_6$ Dialkylpiperazin, 1H-Imidazol, 1-Methylimidazol, 1,2-Dimethylimidazol, 1-Ethylimidazol, 1-Ethyl-2-methylimidazol, 1-Butylimidazol, 1-Butyl-2-methylimidazol, 1-Propylimidazol, 1-iso-Propylimidazol, 1-Allylimidazol, 1-Hexylimidazol, 1-Octylimidazol, 1-Benzylimidazol, 1-Benzyl-2-methylimidazol, Pyridin, 4-(Dimethylamino)-pyridin, Benzotriazol, 1-Methylbenzotriazol, 2-Mercaptobenzothiazol und 1,5-Diazabicyclo[4.3.0]non-5-en (DBN), 1,8-Diazabicyclo[5.4.0]undec-7-en (DBU), 1,4-Diazabicyclo-[2.2.2]-octan (DABCO®), 7-Methyl-1,5,7-triazabicyclo[4.4.0]dec-5-ene (MTBD), 1,8-Bis-(dimethylamino)-naphthalin (Proton Sponge®), N,N,N',N'-Tetramethylethylendiamin (TMEDA), 4,5-Bis-(dimethyl-amino)-fluoren und 1,8-Bis-(hexamethyltriaminophosphazenyl)-naphthalin

**12.** Zusammensetzung nach einem der Ansprüche 1 bis 11, wobei das Anion [$B^-$] der ionischen Flüssigkeit $BF_4^-$ ist.

**13.** Verwendung der Zusammensetzung nach einem der Ansprüche 1 bis 12 als Elektrolyt, Wärmeträger, Kühlmittel, Kraftübertragungsmedium, Schmiermittel, Dichtungsmittel, Lösungsmittel, Medium zum thermischen Quenchen von Metallen, Antistatikum, Flammschutzmittel und/oder Weichmacher.

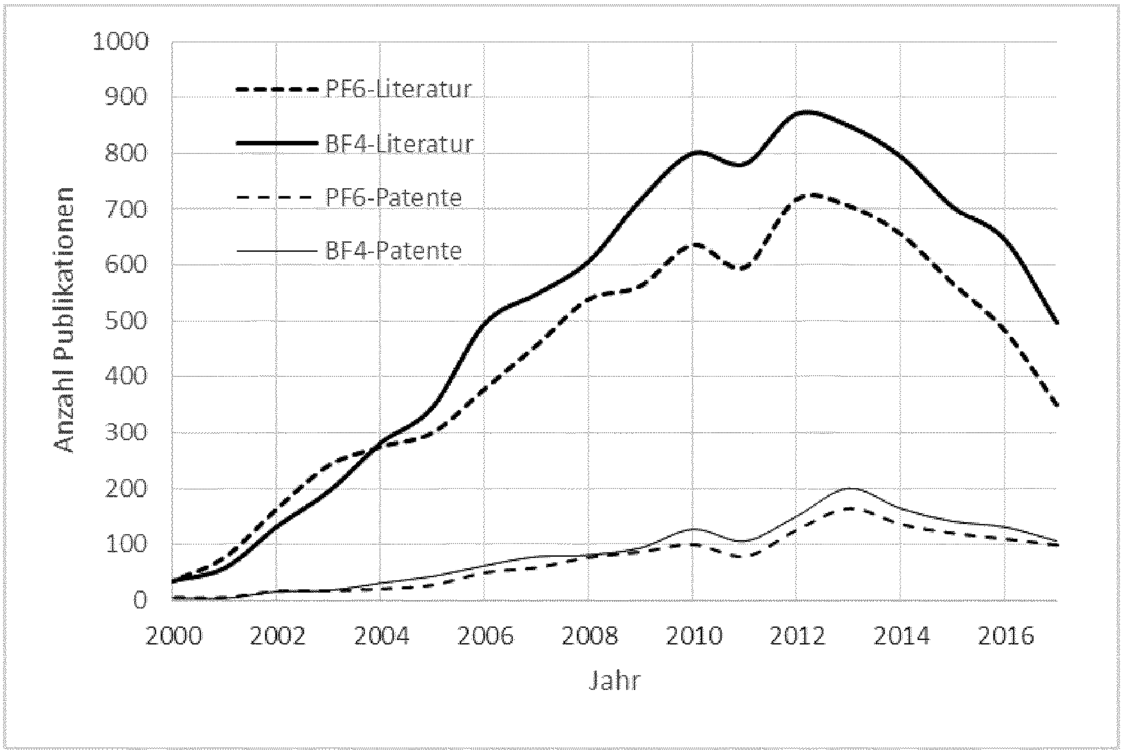
1000
900
800
700
600
500
400
300
200
100
0
Anzahl Publikationen
PF6-Literatur
BF4-Literatur
PF6-Patente
BF4-Patente
2000
2002
2004
2006
2008
2010
2012
2014
2016
Jahr

Fig. 1

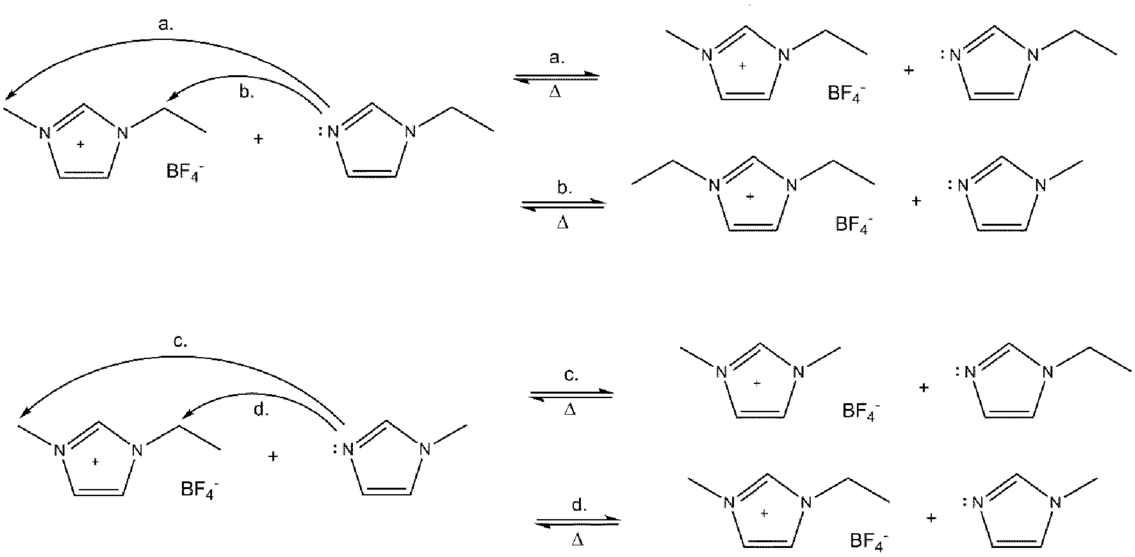
a.
b.
c.
d.
Δ

Fig. 2

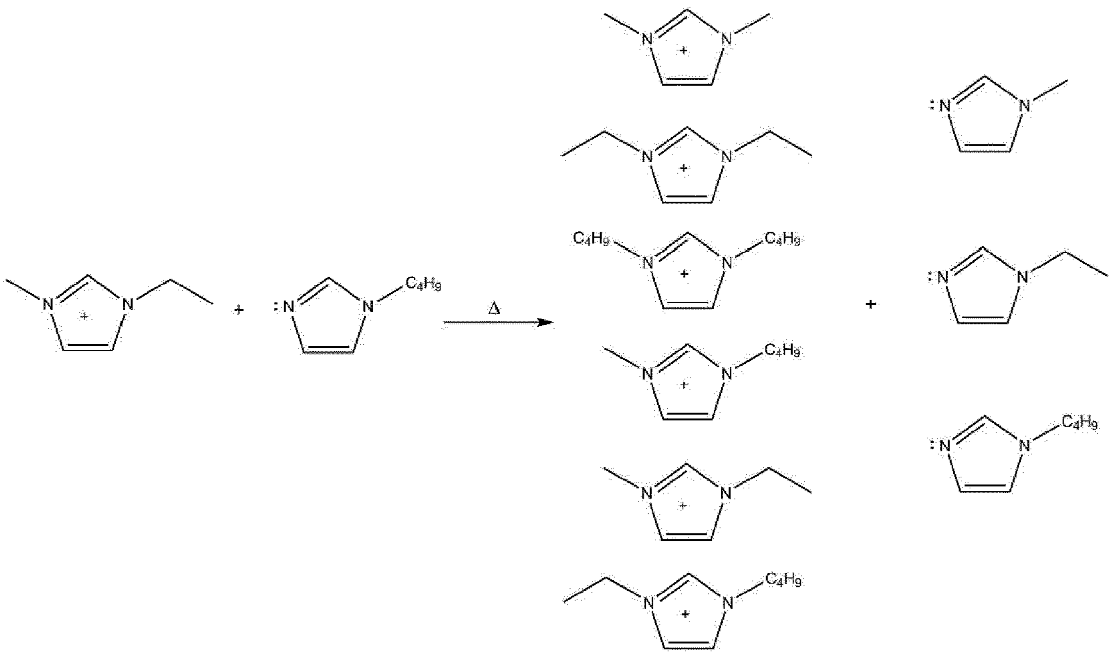
Δ

Fig. 3A

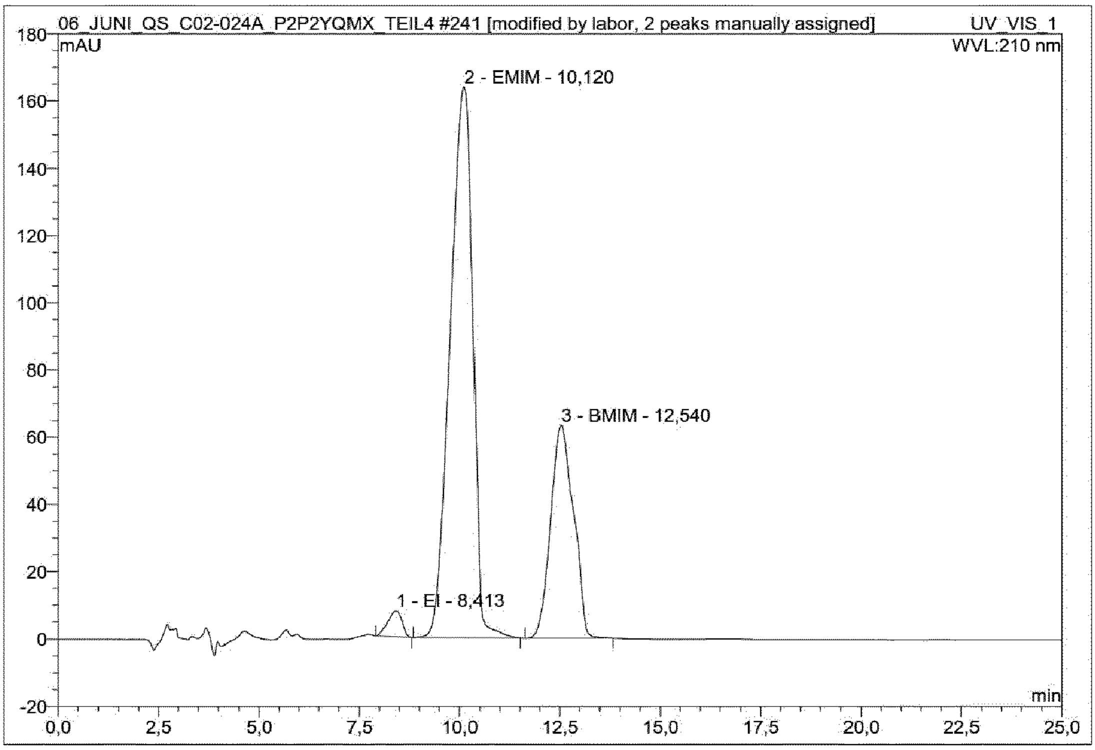
06_JUNI_QS_C02-024A_P2P2YQMX_TEIL4 #241 [modified by labor, 2 peaks manually assigned]
UV_VIS_1
WVL:210 nm
mAU
2 - EMIM - 10,120
3 - BMIM - 12,540
1 - EI - 8,413
180
160
140
120
100
80
60
40
20
0
-20
0,0
2,5
5,0
7,5
10,0
12,5
15,0
17,5
20,0
22,5
25,0
min

Fig. 3B

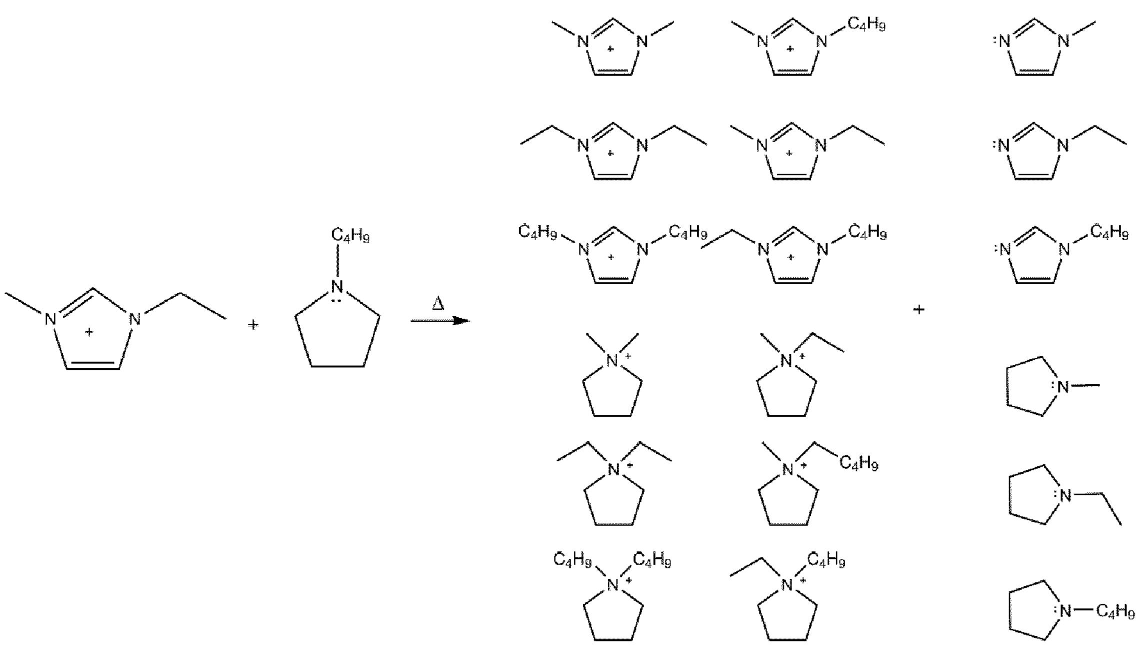


Fig. 4

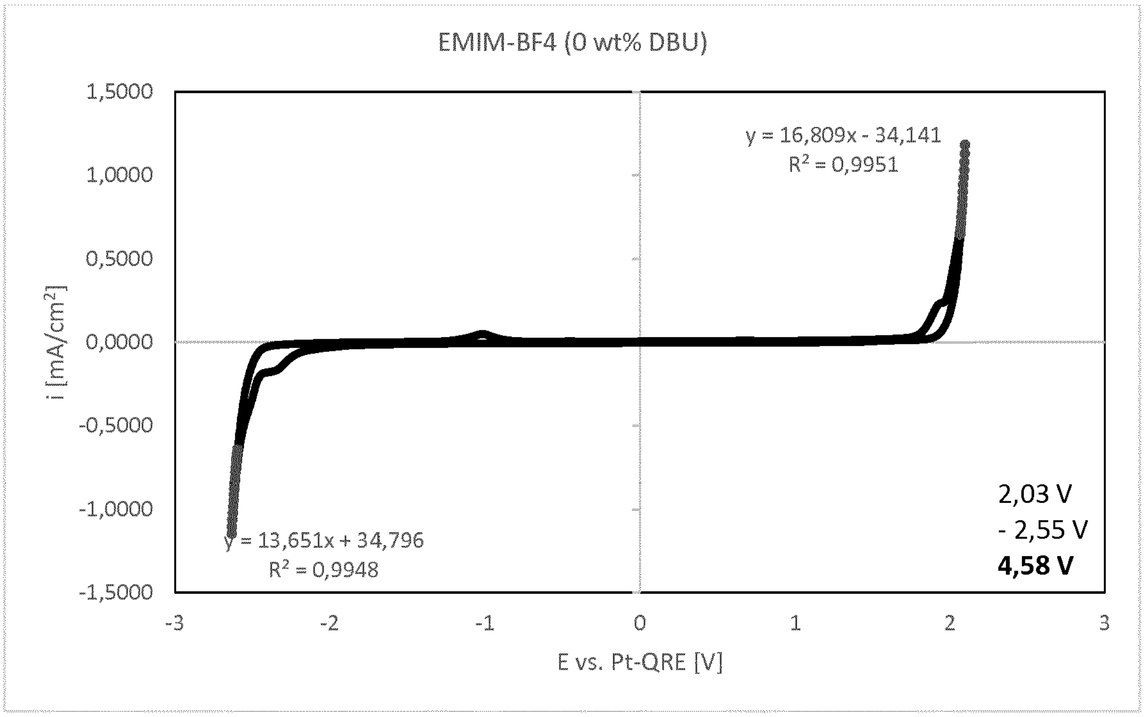
EMIM-BF4 (0 wt% DBU)
1,5000
1,0000
0,5000
0,0000
-0,5000
-1,0000
-1,5000
i [mA/cm2]
y = 16,809x - 34,141
R² = 0,9951
y = 13,651x + 34,796
R² = 0,9948
2,03 V
- 2,55 V
4,58 V
-3
-2
-1
0
1
2
3
E vs. Pt-QRE [V]

Fig. 5

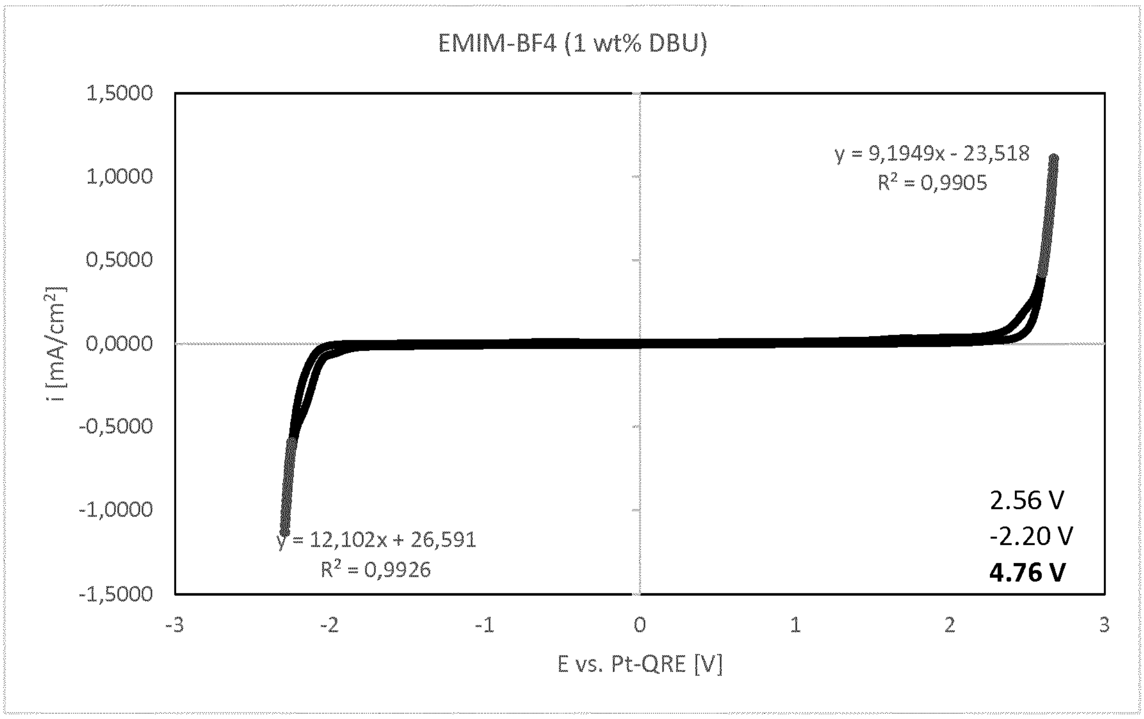
EMIM-BF4 (1 wt% DBU)
1,5000
1,0000
0,5000
0,0000
-0,5000
-1,0000
-1,5000
i [mA/cm2]
y = 9,1949x - 23,518
R² = 0,9905
y = 12,102x + 26,591
R² = 0,9926
2.56 V
-2.20 V
4.76 V
-3
-2
-1
0
1
2
3
E vs. Pt-QRE [V]

Fig. 6

Europäisches Patentamt
European Patent Office
Office européen des brevets

# EUROPÄISCHER RECHERCHENBERICHT

Nummer der Anmeldung
EP 18 19 9151

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2006/120145 A1 (LINDE AG [DE]; KOTSCHAN MICHAEL [AT]; KALB ROLAND [AT]) 16. November 2006 (2006-11-16) * Ansprüche 5, 12, 18 * ----- | 1-13 | INV. C07D233/58 |
| X | ANASTASIA EFIMOVA ET AL: "Thermal Resilience of Imidazolium-Based Ionic Liquids-Studies on Short- and Long-Term Thermal Stability and Decomposition Mechanism of 1-Alkyl-3-methylimidazolium Halides by Thermal Analysis and Single-Photon Ionization Time-of-Flight Mass Spectrometry", JOURNAL OF PHYSICAL CHEMISTRY PART B: CONDENSED MATTER, MATERIALS, SURFACES, INTERFACES & BIOPHYSICAL, Bd. 122, Nr. 37, 22. August 2018 (2018-08-22), Seiten 8738-8749, XP055531332, US ISSN: 1520-6106, DOI: 10.1021/acs.jpcb.8b06416 * Seite 8742, linke Spalte * * Abbildung 1 * ----- | 1-11 | |
| X | XUE H ET AL: "Review of ionic liquids with fluorine-containing anions", JOURNAL OF FLUORINE CHEMISTRY, ELSEVIER, NL, Bd. 127, Nr. 2, 1. Februar 2006 (2006-02-01), Seiten 159-176, XP028044119, ISSN: 0022-1139, DOI: 10.1016/J.JFLUCHEM.2005.11.007 [gefunden am 2006-02-01] * Seite 172, linke Spalte * ----- -/-- | 13 | RECHERCHIERTE SACHGEBIETE (IPC) C07D |

Der vorliegende Recherchenbericht wurde für alle Patentansprüche erstellt

3

| Recherchenort | Abschlußdatum der Recherche | Prüfer |
|---|---|---|
| München | 10. Dezember 2018 | Gutke, Hans-Jürgen |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 18 19 9151

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | RICHARD P. SWATLOSKI ET AL: "Ionic liquids are not always green: hydrolysis of 1-butyl-3-methylimidazolium hexafluorophosphate", GREEN CHEMISTRY, Bd. 5, Nr. 4, 1. Januar 2003 (2003-01-01), Seite 361, XP55531416, GB ISSN: 1463-9262, DOI: 10.1039/b304400a * Seite 362, rechte Spalte, Zeilen 6-7 * ----- | 1,2,13 | |
| X | MIRZAEI YOUSEF R ET AL: "Syntheses of 1-Alkyl-1,2,4-triazoles and the Formation of Quaternary 1-Alkyl-4-polyfluoroalkyl-1,2,4-triazolium Salts Leading to Ionic Liquids", JOURNAL OF ORGANIC CHEMISTRY,, Bd. 67, Nr. 26, 1. Januar 2002 (2002-01-01), Seiten 9340-9345, XP002418350, ISSN: 0022-3263, DOI: 10.1021/JO026350G * Verbindung 5n * * Tabelle 2 * ----- | 1,2,12 | |
| | | | RECHERCHIERTE SACHGEBIETE (IPC) |

Der vorliegende Recherchenbericht wurde für alle Patentansprüche erstellt

3

| Recherchenort | Abschlußdatum der Recherche | Prüfer |
|---|---|---|
| München | 10. Dezember 2018 | Gutke, Hans-Jürgen |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 18 19 9151

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-12-2018

| Im Recherchenbericht angeführtes Patentdokument | Datum der Veröffentlichung | Mitglied(er) der Patentfamilie | Datum der Veröffentlichung |
|---|---|---|---|
| WO 2006120145 A1 | 16-11-2006 | AT 501793 A1 | 15-11-2006 |
| | | AU 2006245832 A1 | 16-11-2006 |
| | | CN 101189437 A | 28-05-2008 |
| | | EP 1877668 A1 | 16-01-2008 |
| | | JP 2008540897 A | 20-11-2008 |
| | | KR 20080009222 A | 25-01-2008 |
| | | US 2008166243 A1 | 10-07-2008 |
| | | WO 2006120145 A1 | 16-11-2006 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2013113461 A, Kalb, Roland
- WO 2010136403 A, Filzwieser, Iris und Filzwieser Andreas


### In der Beschreibung aufgeführte Nicht-Patentliteratur


- Ionic Liquids in Synthesis. Verlag Wiley-VCH, 2008
- **MACFARLANE ; DOUG ; KAR ; MEGA ; PRINGLE ; JENNIFER M.** Fundamentals of Ionic Liquids: From Chemistry to Applications. Verlag Wiley-VCH, 2017
- **FREEMANTLE, M.** *Chem. Eng. News,* 2000, vol. 78, 37
- **XUE, HONG ; VERMA, RAJENDAR ; SHREEVE, JEAN'NE M.** Review of ionic liquids with fluorine-containing anions. *Journal of Fluorine Chemistry,* 2006, vol. 127, 159-176
- **MACFARLANE et al.** *Energy Environ. Sci.,* 2014, vol. 7, 232
- **CHERNIKOVA, E. A et al.** *Russian Chemical Reviews,* 2015, vol. 84 (8), 875-890
- **MEYER, CAROLIN ; WERNER, SEBASTIAN ; HAUMANN, MARCO ; WASSERSCHEID, PETER.** Ionic Liquids Completely UnCOILed. 2015
- **STEUDTE, STEPHANIE ; STOLTE, STEFAN et al.** *Chem. Commun.,* 2012, vol. 48, 9382-9384
- **WILKES, JOHN S. ; ZAWOROTKO, MICHAEL J.** *Journal of the Chemical Society, Chemical Communications,* 1992, vol. 13, 965-7
- **FULLER, JOAN ; CARLIN, RICHARD T. ; DE LONG, HUGH C. ; HAWORTH, DUSTIN.** *Journal of the Chemical Society, Chemical Communications,* 1994, vol. 3, 299-300
- **STEUDTE, STEPHANIE ; STOLTE, STEFAN et al.** Hydrolysis study of fluoroorganic and cyano-based ionic liquid anions - consequences for operational safety and environmental stability. *Green Chem.,* 2012, vol. 14, 2472
- **FREIRE, MARA G. ; COUTINHO, JOAO A. P. et al.** Hydrolysis of Tetrafluoroborate and Hexafluorophosphate Counter Ions in Imidazolium-Based Ionic Liquids. *J. Phys. Chem. A,* 2010, vol. 114, 3744-3749
- **KULCHAT, SIRINAN ; LEHN, JEAN-MARIE.** Dynamic Covalent Chemistry of Nucleophilic Substitution, Component Exchange of Quaternary Ammonium Salts. *Chem. Asian J.,* 2015, vol. 10, 2484-2496
- **BOU MALHAMA, I. ; LETELLIERA, P. ; TURMINE, M.** *Talanta,* 2008, vol. 77, 48-52
- **KALB, R.S. et al.** *Phys. Chem. Chem. Phys.,* 2016, vol. 18, 31904
- **KALB, R.S. et al.** *React. Chem. Eng.,* 2017, vol. 2, 432
- *CHEMICAL ABSTRACTS,* 143314-16-3
- *J. Heterocyclic Chem.,* 2002, vol. 39, 287
- *Thermochimica Acta,* 2006, vol. 449, 67-72
- *Croat. Chem. Acta,* 2014, vol. 87, 385
- *CHEMICAL ABSTRACTS,* 174501-64-5
- *CHEMICAL ABSTRACTS,* 7098-07-9
- **HOLBREY, J.D. ; SEDDON, K.R.** The phase behaviour of 1-alkyl-3-methylimidazolium tetrafluoroborates; ionic liquids and ionic liquid crystals. *J. Chem. Soc., Dalton Trans.,* 1999, 2133-2139
- *CHEMICAL ABSTRACTS,* 4316-42-1
- *CHEMICAL ABSTRACTS,* 767-10-2
- *CHEMICAL ABSTRACTS,* 464927-72-8
- *CHEMICAL ABSTRACTS,* 121-44-8
- *CHEMICAL ABSTRACTS,* 86042-82-2
- *CHEMICAL ABSTRACTS,* 1040887-91-9
- **M. LIPSZTAJN ; R. A. OSTERYOUNG.** *J. Electrochem. Soc.,* 1983, vol. 130 (9), 1968-1969
- *CHEMICAL ABSTRACTS,* 6674-22-2